(19) Europäisches Patentamt
European Patent Office
Office européen des brevets

(11) EP 4 810 065 A1

(12) EUROPEAN PATENT APPLICATION
published in accordance with Art. 153(4) EPC

(43) Date of publication:
**23.09.2026 Bulletin 2026/39**

(21) Application number: **24890762.8**

(22) Date of filing: **14.11.2024**

(51) International Patent Classification (IPC):
*A61K 47/68 (2017.01)* *A61K 31/4375 (2006.01)*
*C07D 401/14 (2006.01)* *C07D 471/04 (2006.01)*
*A61P 35/00 (2006.01)*

(52) Cooperative Patent Classification (CPC):
**A61K 31/4375; A61K 47/68; A61P 35/00; C07D 401/14; C07D 471/04**

(86) International application number:
**PCT/CN2024/132015**

(87) International publication number:
**WO 2025/103407 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.11.2023 CN 202311536729**
**06.08.2024 CN 202411076101**

(71) Applicants:
- **Jiangsu Hansoh Pharmaceutical Group Co., Ltd.**
  **Lianyungang, Jiangsu 222047 (CN)**
- **Shanghai Hansoh Biomedical Company Limited**
  **Pudong New Area, Shanghai 201203 (CN)**
- **Changzhou Hansoh Pharmaceutical Company Limited**
  **Changzhou, Jiangsu 213001 (CN)**

(72) Inventors:
- **LIU, Junhao**
  **Shanghai 201203 (CN)**
- **SUN, Danni**
  **Shanghai 201203 (CN)**
- **ZHOU, Yuanfeng**
  **Shanghai 201203 (CN)**

(74) Representative: **Hanby, Abigail Rose**
**GSK**
**Legal & Compliance - Global Patents**
**79 New Oxford Street**
**London WC1A 1DG (GB)**

# (54) COMBINED USE OF ANTIBODY-DRUG CONJUGATE AND POLY(ADENOSINE DIPHOSPHATE RIBOSE) POLYMERASE INHIBITOR

(57) The present invention relates to the combined use of an antibody-drug conjugate and a poly(adenosine diphosphate ribose) polymerase inhibitor. Specifically, provided is the use of an antibody-drug conjugate or a pharmaceutically acceptable salt, metabolite or solvate thereof individually or combined with a poly(adenosine diphosphate ribose) polymerase inhibitor in the preparation of drugs for preventing and/or treating cancers.

MX-1 Xenograft
Tumor volume (mm³, Mean ± SEM)
Time after administration (days)
G1. Vehicle control (0.5% HPMC), p.o. QD
G2. Drug B, 0.3 mg/kg, p.o. QD
G3. Drug A 1 mg/kg, i.v. Single dose
G4. Drug A 2 mg/kg, i.v. Single dose
G5. Drug B, 0.3 mg/kg + Drug A, 1 mg/kg
G6. Drug B, 0.3 mg/kg + Drug A, 2 mg/kg

Fig. 4



Processed by Luminess, 75001 PARIS (FR)

## Description

## Technical Field

The present application belongs to the field of medicine, and relates to the use of an antibody-drug conjugate, alone or in combination, in the preparation of a medication for preventing and/or treating cancer. Specifically, the present invention provides the use of an anti-B7 homolog 4 antibody-drug conjugate, or a pharmaceutically acceptable salt, metabolite, or solvate thereof, alone or in combination with a poly(ADP-ribose) polymerase inhibitor, in the preparation of a medication for preventing and/or treating cancer.

## Background Art

An antibody-drug conjugate (ADC) is a class of targeted biologics in which a cytotoxic drug is linked to a monoclonal antibody through a linker, with the monoclonal antibody serving as a carrier to efficiently deliver the small-molecule cytotoxic drug to target tumor cells in a targeted manner. Tumor-specific antibodies enable ADC drugs to selectively deliver small-molecule cytotoxic drugs, while reducing the off-target effects of the small-molecule cytotoxic drugs and retaining their anti-tumor properties, thereby effectively improving the benefit-risk ratio of anti-tumor treatment. B7 homolog 4 is a newly discovered member of the B7 family and plays an important role in multiple cellular biological processes, such as cell differentiation, proliferation, and apoptosis, and may affect tumor-cell invasion and metastasis; meanwhile, the B7 family comprises important costimulatory molecules that can affect processes such as T-cell proliferation and B-cell activation. Studies have shown that B7 homolog 4 is highly expressed in various tumors, such as cholangiocarcinoma, breast cancer, endometrial cancer, non-small cell lung cancer, ovarian cancer, gastric cancer, and pancreatic cancer, while its expression in normal tissues is limited; therefore, B7 homolog 4 is expected to be a promising target for ADCs.

A poly(ADP-ribose) polymerase (poly adenosinediphosphate ribose polymerase, PARP) inhibitor causes the accumulation of single-strand damage and triggers DNA double-strand breaks by inhibiting PARP1-mediated repair of DNA single-strand damage. In tumors deficient in homologous recombination repair (Homologous Recombination Repair, HRR), DNA double-strand breaks cannot be accurately repaired, thereby producing a "synthetic lethality" effect, causing the PARP inhibitor to produce single-drug anti-tumor activity.

Based on the mechanism of action of PARP inhibitors, combined use of PARP inhibitors and other DNA-damaging drugs can significantly increase efficacy; on the other hand, PARP inhibitors and other DNA-damaging drugs have overlapping toxic side effects, including leukopenia, anemia, and the like, which limit the application of such combination therapies. Therefore, use of an ADC having a DNA-damaging drug as a payload in combination with a PARP inhibitor can reduce drug exposure in normal tissues, improve efficacy, and at the same time avoid overlapping toxic side effects.

## Summary of the Invention

The present disclosure provides the use of an antibody-drug conjugate in combination with a poly(ADP-ribose) polymerase inhibitor in the preparation of a medication for treating cancer, wherein the structure of the antibody-drug conjugate is shown in formula (I):

(I)

In the formula, n is 1 to 10, preferably 2 to 8, and more preferably 3 to 8, and n is a decimal or an integer; Pc is an anti-B7H4 antibody or an antigen-binding fragment thereof.

In some embodiments, the anti-B7H4 antibody or antigen-binding fragment thereof described in the present disclosure comprises heavy-chain HCDR1, HCDR2, and HCDR3 having the amino acid sequences set forth in SEQ ID

NOs: 01, 02, and 03, respectively, and light-chain LCDR1, LCDR2, and LCDR3 having the amino acid sequences set forth in SEQ ID NOs: 04, 05, and 06, respectively.

The above-described CDR sequences are shown in the following table:

Table 1 CDR sequences of each heavy chain and light chain

| HCDR1 | NYYMS SEQ ID NO:01 | LCDR1 | RASQSISDYLH SEQ ID NO:04 |
|---|---|---|---|
| HCDR2 | YVSSGGGSTYYSDSVKG SEQ ID NO:02 | LCDR2 | FASQSIS SEQ ID NO:05 |
| HCDR3 | ESYSQGNYFDY SEQ ID NO:03 | LCDR3 | QNGHSFSLT SEQ ID NO:06 |
| Note: The CDR sequences are defined according to the Kabat rules. | | | |

In some embodiments, the anti-B7H4 antibody or antigen-binding fragment thereof described in the present disclosure is a humanized antibody or a fragment thereof.

In some embodiments, the anti-B7H4 antibody or antigen-binding fragment thereof described in the present disclosure comprises a heavy-chain constant region of a human IgG1, IgG2, IgG3, or IgG4 isotype, and a light-chain constant region comprising κ or λ; preferably, the anti-B7H4 antibody or antigen-binding fragment thereof comprises a heavy-chain constant region of an IgG1 or IgG4 isotype.

In some embodiments, the heavy-chain variable region sequence of the anti-B7H4 antibody or antigen-binding fragment thereof described in the present disclosure is the sequence set forth in SEQ ID NO: 07 or a variant thereof, and the light-chain variable region sequence is the sequence set forth in SEQ ID NO: 08 or a variant thereof.

The sequences of the heavy-chain and light-chain variable regions of the aforementioned anti-B7H4 antibody or antigen-binding fragment thereof are as follows:

Heavy-chain variable region sequence

*EVQLVESGGGLVQPGGSLRLSCAASGFTFS*<u>NYYMS</u>*WVRQAPGKGLEWVA*<u>YVSSGGGSTYYSDSVKG</u>*RFTISRDNAKNTLYLQMSSLRAEDTAVYYCTR*<u>ESYSQGNYFD</u>*YWGQGTTVTVSS*

SEQ ID NO: 07

Light-chain variable region sequence

*EIVMTQSPATLSLSPGERATLSC*<u>RASQSISDYLH</u>*WYQQKPGQSPRLLIK*<u>FASQSIS</u>*GIPARFSGSGSGTDFTLTISSLEPEDFAVYYC*<u>QNGHSFSLT</u>*FGQGTKLEIK*

SEQ ID NO: 08

Note: The order is FR1-CDR1-FR2-CDR2-FR3-CDR3-FR4; italicized parts in the sequence are FR sequences, and underlined parts are CDR sequences, wherein the CDR sequences are defined according to the Kabat rules.

In some embodiments, the heavy-chain sequence of the anti-B7H4 antibody or antigen-binding fragment thereof described in the present disclosure is the sequence as shown in SEQ ID NO: 09 or a variant thereof, and the light-chain sequence is the sequence as shown in SEQ ID NO: 10 or a variant thereof.

The sequences of the heavy chain and the light chain of the aforementioned anti-B7H4 antibody or antigen-binding fragment thereof are as follows:

Heavy-chain (IgG1) amino acid sequence: (SEQ ID NO: 09)

EVQLVESGGGLVQPGGSLRLSCAASGFTFSNYYMSWVRQAPGKGLEWVAYVSS GGGSTYYSDSVKGRFTISRDNAKNTLYLQMSSLRAEDTAVYYCTRESYSQGNYFDY WGQGTTVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCDK THTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQPENNYKTT PPVLDSDGSFFLYSKLTVDKSRWQQGNVFSCSVMHEALHNHYTQKSLSLSPGK

Light-chain (λ) amino acid sequence: (SEQ ID NO: 10)

EIVMTQSPATLSLSPGERATLSCRASQSISDYLHWYQQKPGQSPRLLIKFASQSISG IPARFSGSGSGTDFTLTISSLEPEDFAVYYCQNGHSFSLTFGQGTKLEIKRTVAAPSVFIF PPSDEQLKSGTASVVCLLNNFYPREAKVQWKVDNALQSGNSQESVTEQDSKDSTYSL SSTLTLSKADYEKHKVYACEVTHQGLSSPVTKSFNRGEC

In some embodiments, the poly(ADP-ribose) polymerase inhibitor is selected from one or more of Olaparib, Fluzoparib, Niraparib, Pamiparib, Rucaparib, Talazoparib, Veliparib, Senaparib, CEP-8983, BGB-290, or 1'-((7-ethyl-6-oxo-5,6-dihydro-1,5-naphthyridin-3-yl)methyl)-N-methyl-1',2',3',6'-tetrahydro-[3,4'-bipyridine]-6-carboxamide.

The combination described in the present disclosure has added or synergistic effects.

In optional embodiments, the antibody-drug conjugate and the poly(ADP-ribose) polymerase inhibitor are respectively contained as active ingredients in different formulations and are administered simultaneously, sequentially, continuously, alternately, or separately.

On the other hand, the present application discloses a use of the antibody-drug conjugate and the poly(ADP-ribose) polymerase inhibitor further in combination with an anti-VEGF antibody in the preparation of a medication for treating cancer.

In optional embodiments, the above VEGF antibody is selected from Bevacizumab, Ranibizumab, Sevacizumab, Suvemcitug, Varisacumab, CMAB-801, and LYN-00101.

In optional embodiments, the antibody-drug conjugate, the poly(ADP-ribose) polymerase inhibitor, and the anti-VEGF antibody are respectively contained as active ingredients in different formulations, and are administered simultaneously, in parallel, sequentially, continuously, alternately, or separately.

In another aspect, the aforementioned antibody-drug conjugate and poly(ADP-ribose) polymerase inhibitor are contained as active ingredients in a single formulation and then administered.

In another aspect, the above antibody-drug conjugate, poly(ADP-ribose) polymerase inhibitor, and anti-VEGF antibody are contained as active ingredients in a single formulation and then administered.

In some embodiments, the cancer is a solid tumor, and preferably, the cancer is selected from uterine cancer, breast cancer, ovarian cancer, pancreatic cancer, cholangiocarcinoma, fallopian tube cancer, gastric cancer, colorectal cancer, primary peritoneal cancer, prostate cancer, and lung cancer; more preferably it is uterine cancer, breast cancer, or ovarian cancer.

Further, the cancer is selected from cervical cancer, endometrial cancer, triple-negative breast cancer, epithelial ovarian cancer, platinum-sensitive recurrent ovarian cancer, intrahepatic cholangiocarcinoma, and extrahepatic cholangiocarcinoma; preferably endometrial cancer, triple-negative breast cancer, and ovarian epithelial cancer; further preferably, the ovarian epithelial cancer is selected from high-grade serous ovarian cancer, low-grade serous ovarian cancer, endometrioid ovarian cancer, mucinous ovarian cancer, and ovarian clear cell carcinoma.

In optional embodiments, the dose of the antibody-drug conjugate is 0.1 mg/kg to 20.0 mg/kg, preferably 1.0 mg/kg to 15.0 mg/kg; more preferably 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, 10.2 mg/kg, 10.4 mg/kg, 10.6 mg/kg, 10.8 mg/kg, 11.0 mg/kg, 11.2 mg/kg, 11.4 mg/kg, 11.6 mg/kg, 11.8 mg/kg, 12.0 mg/kg, 12.2 mg/kg, 12.4 mg/kg, 12.6 mg/kg, 12.8 mg/kg, 13.0

mg/kg, 13.2 mg/kg, 13.4 mg/kg, 13.6 mg/kg, 13.8 mg/kg, 14.0 mg/kg, 14.2 mg/kg, 14.4 mg/kg, 14.6 mg/kg, 14.8 mg/kg, 15.0 mg/kg, or any value between two values thereof.

In optional embodiments, the administration frequency of the antibody-drug conjugate is once every week, once every two weeks, once every three weeks, or once every four weeks.

In optional embodiments, the administration dose of the poly(ADP-ribose) polymerase inhibitor is 1-500 mg/kg, preferably 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 110 mg/kg, 120 mg/kg, 130 mg/kg, 140 mg/kg, 150 mg/kg, 160 mg/kg, 170 mg/kg, 180 mg/kg, 190 mg/kg, 200 mg/kg, 210 mg/kg, 220 mg/kg, 230 mg/kg, 240 mg/kg, 250 mg/kg, 260 mg/kg, 270 mg/kg, 280 mg/kg, 290 mg/kg, 300 mg/kg, 320 mg/kg, 340 mg/kg, 350 mg/kg, 360 mg/kg, 380 mg/kg, 400 mg/kg, 420 mg/kg, 440 mg/kg, 450 mg/kg, 460 mg/kg, 480 mg/kg, or 500 mg/kg, or any value between two values thereof.

In preferred embodiments, the administration dose of the poly(ADP-ribose) polymerase inhibitor is 10-300 mg/kg, more preferably 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 110 mg/kg, 120 mg/kg, 130 mg/kg, 140 mg/kg, 150 mg/kg, 160 mg/kg, 170 mg/kg, 180 mg/kg, 190 mg/kg, 200 mg/kg, 210 mg/kg, 220 mg/kg, 230 mg/kg, 240 mg/kg, 250 mg/kg, 260 mg/kg, 270 mg/kg, 280 mg/kg, 290 mg/kg, 300 mg/kg, or any value between two values thereof.

In optional embodiments, the administration frequency of the poly(ADP-ribose) polymerase inhibitor is once daily, twice daily, three times daily, once every two days, or once every three days.

In optional embodiments, the dose of the anti-VEGF antibody is 1.0 mg/kg to 100 mg/kg, preferably 1.0 mg/kg to 40 mg/kg, more preferably 1.0 mg/kg to 30 mg/kg, and further preferably 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 3.2 mg/kg, 3.4 mg/kg, 3.6 mg/kg, 3.8 mg/kg, 4.0 mg/kg, 4.2 mg/kg, 4.4 mg/kg, 4.6 mg/kg, 4.8 mg/kg, 5.0 mg/kg, 5.2 mg/kg, 5.4 mg/kg, 5.6 mg/kg, 5.8 mg/kg, 6.0 mg/kg, 6.2 mg/kg, 6.4 mg/kg, 6.6 mg/kg, 6.8 mg/kg, 7.0 mg/kg, 7.2 mg/kg, 7.4 mg/kg, 7.6 mg/kg, 7.8 mg/kg, 8.0 mg/kg, 8.2 mg/kg, 8.4 mg/kg, 8.6 mg/kg, 8.8 mg/kg, 9.0 mg/kg, 9.2 mg/kg, 9.4 mg/kg, 9.6 mg/kg, 9.8 mg/kg, 10.0 mg/kg, 10.2 mg/kg, 10.4 mg/kg, 10.6 mg/kg, 10.8 mg/kg, 11.0 mg/kg, 11.2 mg/kg, 11.4 mg/kg, 11.6 mg/kg, 11.8 mg/kg, 12.0 mg/kg, 12.2 mg/kg, 12.4 mg/kg, 12.6 mg/kg, 12.8 mg/kg, 13.0 mg/kg, 13.2 mg/kg, 13.4 mg/kg, 13.6 mg/kg, 13.8 mg/kg, 14.0 mg/kg, 14.2 mg/kg, 14.4 mg/kg, 14.6 mg/kg, 14.8 mg/kg, 15.0 mg/kg, 15.2 mg/kg, 15.4 mg/kg, 15.6 mg/kg, 15.8 mg/kg, 16.0 mg/kg, 16.2 mg/kg, 16.4 mg/kg, 16.6 mg/kg, 16.8 mg/kg, 17.0 mg/kg, 17.2 mg/kg, 17.4 mg/kg, 17.6 mg/kg, 17.8 mg/kg, 18.0 mg/kg, 18.2 mg/kg, 18.4 mg/kg, 18.6 mg/kg, 18.8 mg/kg, 19.0 mg/kg, 19.2 mg/kg, 19.4 mg/kg, 19.6 mg/kg, 19.8 mg/kg, 20.0 mg/kg, 20.2 mg/kg, 20.4 mg/kg, 20.6 mg/kg, 20.8 mg/kg, and 30.0 mg/kg.

In optional embodiments, the administration frequency of the anti-VEGF antibody is once every week, once every two weeks, or once every three weeks.

In preferred embodiments, the dose of the anti-VEGF antibody is 15 mg/kg, and the administration frequency is once every three weeks.

The present disclosure further provides a pharmaceutical composition containing the above-described antibody-drug conjugate and the poly(ADP-ribose) polymerase inhibitor, comprising one or more pharmaceutically acceptable excipients, diluents, or carriers.

The present disclosure further provides a pharmaceutical composition containing the above-described antibody-drug conjugate, poly(ADP-ribose) polymerase inhibitor, and anti-VEGF antibody, comprising one or more pharmaceutically acceptable excipients, diluents, or carriers.

In addition, the pharmaceutical composition of the present disclosure may also be administered to a patient or subject in need of such treatment by any suitable mode of administration, such as oral, parenteral, rectal, pulmonary, topical administration, subcutaneous injection, intramuscular injection, intravenous injection, and the like.

The present disclosure further provides a method for treating cancer, comprising: administering in combination to a patient an effective amount of the antibody-drug conjugate as shown in formula (I) and the poly(ADP-ribose) polymerase inhibitor, wherein the combination administration may be simultaneous, parallel, sequential, continuous, alternating, or separate administration.

The present disclosure further provides a method for treating cancer, comprising: administering in combination to a patient an effective amount of the antibody-drug conjugate as shown in formula (I), the poly(ADP-ribose) polymerase inhibitor, and the anti-VEGF antibody, wherein the combination administration may be simultaneous, parallel, sequential, continuous, alternating, or separate administration.

The present disclosure further provides a method for treating or preventing cancer, the method comprising administering in combination to a subject in need, during the induction treatment stage, the above antibody-drug conjugate, the anti-VEGF antibody, and a platinum drug, wherein the combination administration may be simultaneous, parallel, sequential, continuous, alternating, or separate administration, and during the maintenance treatment stage after the induction treatment, administering in combination to a subject in need the above antibody-drug conjugate, the anti-VEGF antibody, and the poly(ADP-ribose) polymerase inhibitor, wherein the combination administration may be simul-

taneous, parallel, sequential, continuous, alternating, or separate administration.

In optional embodiments, the platinum drug is selected from carboplatin, cisplatin, oxaliplatin, nedaplatin (Nedaplatin), lobaplatin (Lobaplatin), satraplatin (Satraplatin), cycloplatin (Cycloplatin), miboplatin (Miboplatin), Enloplatin, Iproplatin, and Dicycloplatin; preferably carboplatin and/or cisplatin.

In optional embodiments, the administration dose of the platinum drug is 10 mg/m$^2$ to 500 mg/m$^2$, preferably 10 mg/m$^2$ to 200 mg/m$^2$, more preferably 25 mg/m$^2$, 50 mg/m$^2$, 75 mg/m$^2$, 100 mg/m$^2$, 125 mg/m$^2$, 150 mg/m$^2$, 175 mg/m$^2$, or 200 mg/m$^2$, and the administration frequency is once every week, once every two weeks, once every three weeks, or once every four weeks.

In optional embodiments, the administration dose of the platinum drug is calculated by area under the curve (AUC), and is 1 to 20 mg/ml/min, preferably 1 to 10 mg/ml/min, more preferably 2 mg/ml/min, 3 mg/ml/min, 4 mg/ml/min, 5 mg/ml/min, 6 mg/ml/min, 7 mg/ml/min, 8 mg/ml/min, or 9 mg/ml/min, and the administration frequency is once every week, once every two weeks, once every three weeks, or once every four weeks.

In optional embodiments, the platinum drug is administered for up to 6 cycles.

In preferred embodiments, the administration dose of the platinum drug is: cisplatin 75 mg/m$^2$ or carboplatin AUC 5 mg/ml/min by intravenous infusion, and the administration frequency is once every three weeks.

In some embodiments, the cancer is a solid tumor, and preferably, the cancer is selected from uterine cancer, breast cancer, ovarian cancer, pancreatic cancer, cholangiocarcinoma, fallopian tube cancer, gastric cancer, colorectal cancer, primary peritoneal cancer, prostate cancer, and lung cancer; more preferably it is uterine cancer, breast cancer, or ovarian cancer.

In some embodiments, the cancer is selected from cervical cancer, endometrial cancer, triple-negative breast cancer, ovarian epithelial cancer, platinum-sensitive recurrent ovarian cancer, intrahepatic cholangiocarcinoma, and extrahepatic cholangiocarcinoma; preferably it is endometrial cancer, triple-negative breast cancer, or ovarian epithelial cancer; further preferably, the ovarian epithelial cancer is selected from high-grade serous ovarian cancer, low-grade serous ovarian cancer, high-grade endometrioid epithelial ovarian cancer, endometrioid ovarian cancer, mucinous ovarian cancer, and ovarian clear cell carcinoma.

On the other hand, the present disclosure provides the aforementioned anti-B7H4 antibody-drug conjugate for use in treating cancer, wherein the anti-B7H4 antibody-drug conjugate is used in combination with the aforementioned poly(ADP-ribose) polymerase inhibitor.

On the other hand, the present disclosure provides the aforementioned poly(ADP-ribose) polymerase inhibitor for use in treating cancer, wherein the poly(ADP-ribose) polymerase inhibitor is used in combination with an anti-B7H4 antibody-drug conjugate.

In the present disclosure, the so-called "combination" is a mode of administration, which includes various situations in which two or more drugs are administered successively or simultaneously.

Modes of administration of simultaneous administration, separate formulation and co-administration, or separate formulation and successive administration all belong to the combination administration described in the present disclosure. The so-called "simultaneous" here means that at least one dose of the poly(ADP-ribose) polymerase inhibitor and the anti-B7H4 antibody-drug conjugate is given within a certain period of time, optionally two or more drugs being given within 3 days, within 2 days, or within 1 day, wherein the two or more substances all exhibit pharmacological effects. The so-called "successive" administration includes situations in which the poly(ADP-ribose) polymerase inhibitor and the anti-B7H4 antibody-drug conjugate are respectively given in different administration cycles. The time period may be within one administration cycle, optionally within 4 weeks, within 3 weeks, within 2 weeks, or within 1 week. Such a period includes treatment in which the poly(ADP-ribose) polymerase inhibitor and the anti-B7H4 antibody-drug conjugate are given by the same route of administration or by different routes of administration.

Terms

For easier understanding of the present disclosure, certain technical and scientific terms are specifically defined below. Unless otherwise explicitly defined herein, all other technical and scientific terms used herein have the meanings commonly understood by a person of ordinary skill in the art to which the present disclosure belongs.

The present disclosure incorporates the entire contents of application WO2020244657 into the present application.

The term "antibody-drug conjugate" refers to an antibody being linked to a biologically active drug through a stable linking unit. In the present disclosure, "antibody-drug conjugate" refers to linking a monoclonal antibody or an antibody fragment to a biologically active toxic drug through a stable linking unit.

The term "antibody"refers to an immunoglobulin, which is a tetrapeptide-chain structure formed by two identical heavy chains and two identical light chains linked by interchain disulfide bonds. The amino acid composition and arrangement order of the constant region of the immunoglobulin heavy chain are different, and therefore their antigenicity is also different. Accordingly, immunoglobulins may be divided into five classes, or called immunoglobulin isotypes,

namely IgM, IgD, IgG, IgA and IgE, and the corresponding heavy chains thereof are respectively $\mu$ chain, $\delta$ chain, $\gamma$ chain, $\alpha$ chain, and $\varepsilon$ chain. The same class of Ig may further be divided into different subclasses according to differences in the amino acid composition of its hinge region and the number and positions of heavy-chain disulfide bonds, for example IgG may be divided into IgG1, IgG2, IgG3, IgG4. Light chains are divided according to differences in the constant region into $\kappa$ chains or $\lambda$ chains. In the five classes of Ig, each class of Ig may have a $\kappa$ chain or a $\lambda$ chain.

The sequences of about N-terminal 110 amino acids near the Fv region of the antibody heavy chain and light chain vary greatly and are variable regions (C region); the remaining amino acid sequences near the C terminus are relatively stable and are constant regions. The variable region includes 3 hypervariable regions (HVR) and 4 framework regions with relatively conserved sequences (FR). The 3 hypervariable regions determine the specificity of the antibody, and are also called complementarity-determining regions (CDR) . Each light-chain variable region (LCVR) and heavy-chain variable region (HCVR) is composed of 3 CDR regions and 4 FR regions, arranged in sequence from the amino terminus to the carboxyl terminus as follows: FR1, CDR1, FR2, CDR2, FR3, CDR3, FR4. The 3 CDR regions of the light chain refer to LCDR1, LCDR2, and LCDR3; the 3 CDR regions of the heavy chain refer to HCDR1, HCDR2 and HCDR3.

In the present disclosure, the amino acid sequences of the above-mentioned CDRs are all as shown according to the Kabat definition rules. However, it is well known to those skilled in the art that in the art the CDR of an antibody may be defined by a variety of methods, for example Chothia based on the three-dimensional structure of the antibody and the topology of the CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), the international ImMunoGeneTics database (IMGT) (World Wide Web imgt.cines.fr/), and the North CDR definition based on affinity propagation clustering using a large number of crystal structures. Those skilled in the art will appreciate that, unless otherwise specified, the terms "CDR" and "complementarity-determining region" of a given antibody or region thereof (for example, a variable region) should be understood as encompassing complementarity-determining regions as defined according to any of the known schemes described herein. Although the scope of protection sought by the present invention is based on the sequences defined according to the Kabat rules, amino acid sequences corresponding to other CDR definition rules should also fall within the scope of the present invention.

The term "antigen-binding fragment" refers to one or more fragments of an antibody that retain the ability to specifically bind an antigen. It has been shown that fragments of a full-length antibody can be used to perform the antigen-binding function of the antibody. Examples of binding fragments included in the "antigen-binding fragment" include (i) a Fab fragment, a monovalent fragment composed of VL, VH, CL, and CH1 domains; (ii) an $F(ab')_2$ fragment, a bivalent fragment containing two Fab fragments linked by a disulfide bridge on the hinge region; (iii) an Fd fragment composed of VH and CH1 domains; (iv) an Fv fragment composed of the VH and VL domains of a single arm of an antibody; (v) a single-domain or dAb fragment (Ward et al., (1989) Nature 341: 544-546), which is composed of a VH domain; and (vi) an isolated complementarity-determining region (CDR) or (vii) a combination of two or more isolated CDRs optionally linked by a synthetic linker.

The term "drug load" refers to the average number of cytotoxic drugs loaded on each ligand in the formula (I) molecule, and may also be expressed as the ratio of drug amount to antibody amount, and the range of drug load may be that each antibody (Pc) is linked to 0-12, preferably 1-10, cytotoxic drugs (D). In the embodiments of the present disclosure, the drug load is expressed as n, and may also be referred to as the DAR value, exemplary values being averages of 1, 2, 3, 4, 5, 6, 7, 8, 9, and 10. Conventional methods such as UV/visible spectroscopy, mass spectrometry, ELISA, and HPLC may be used to characterize the average number of drug molecules per ADC molecule after the coupling reaction.

The term "pharmaceutical composition" refers to a product containing one or more active ingredients (for example, an antibody or an ADC) in optionally specified amounts, as well as any product directly or indirectly produced by combining one or more active ingredients in optionally specified amounts. Different active ingredients in the pharmaceutical composition may be independently administered respectively in the form of separate formulations, including administration simultaneously or at different time points to achieve combined enhancement of efficacy. In the present disclosure, "pharmaceutical composition" and "formulation" are not mutually exclusive.

The term "treatment" means administering to a patient a therapeutic agent internally or externally, for example a composition containing any binding compound of the present disclosure, wherein the patient has one or more disease symptoms, and the therapeutic agent is known to have a therapeutic effect on these symptoms. Generally, the therapeutic agent is administered to the treated patient or population in an amount effective to alleviate one or more disease symptoms, so as to induce regression of such symptoms or inhibit the development of such symptoms to any clinically measurable extent. The amount of a therapeutic agent effective to alleviate any specific disease symptom (also referred to as a "therapeutically effective amount") may vary according to a variety of factors, for example the disease state, age, and body weight of the patient, and the ability of the drug to produce the desired efficacy in the patient. Whether the disease symptom has been alleviated may be evaluated by any clinical testing method commonly used by a physician or other healthcare professionals to evaluate the severity or progression status of the symptom. Although the embodiments of the

present disclosure (for example, treatment methods or articles) may be ineffective in alleviating each target disease symptom, as determined according to any statistical testing method known in the art, such as the Student t test, chi-square test, the U test according to Mann and Whitney, Kruskal-Wallis test (H test), Jonckheere-Terpstra test, and Wilcoxon test, they should alleviate the target disease symptom in a statistically significant number of patients.

## Description of the Drawings

Figure 1 Combination index of drug A in combination with drug B for inhibiting proliferation of 7 ovarian cancer cell lines.
Figure 2 Combination index of drug A in combination with drug B for inhibiting proliferation of 4 breast cancer cell lines.
Figure 3 Combination index of drug A in combination with drug B for inhibiting proliferation of 8 endometrial cancer cell lines.
Figure 4 Efficacy of drug A in combination with drug B in a human breast cancer MX-1 subcutaneous xenograft model.
Figure 5 Effect of drug A in combination with drug B on the body weight of MX-1 human breast cancer xenograft-bearing mice.
Figure 6 Efficacy of drug A in combination with drug B in the OV0243 human ovarian cancer PDX subcutaneous xenograft model.
Figure 7 Effect of drug A in combination with drug B on the body weight of OV0243 human ovarian cancer PDX-bearing mice.
Figure 8 Efficacy of drug A in combination with drug B in the RL95-2 human endometrial cancer subcutaneous xenograft model.
Figure 9 Effect of drug A in combination with drug B on the body weight of RL95-2 human endometrial cancer xenograft-bearing mice.

## Specific Embodiments

The present application will be explained in more detail below in conjunction with the examples, and the examples of the present application are only used to illustrate the technical solutions of the present application, and are not intended to limit the substance and scope of the present application.

**Example 1.** Preparation of the anti-B7H4 antibody-drug conjugate and PARP inhibitor

According to the production method described in WO2020244657, hu2F7 (an anti-B7H4 antibody) and an exatecan analog were used to prepare the anti-B7H4 antibody-drug conjugate shown in the following structure, with an average value of y = 6.1 as determined by HIC. Therein, the heavy-chain sequence of hu2F7 is as shown in SEQ ID NO: 09, and the light-chain sequence is as shown in SEQ ID NO: 10. The anti-B7H4 antibody-drug conjugate was used as drug A.

1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was prepared by the method disclosed in WO2022223025, and was used as drug B.

**Example 2.** Evaluation of the inhibitory effect of combined use of drug A and drug B on the proliferation of human ovarian cancer cells, human breast cancer cells, and human endometrial cancer cells.

### 1. Experimental materials

### 1.1 Cell lines and experimental reagents

Human ovarian cancer cells OAW42, KURAMOCHI, CAOV3, COV362, and OAW28; human breast cancer cells HCC38, MX-1, CAL-148, and CAL-51; and human endometrial cancer cells EN, RL95-2, Ishilawa, EFE184, MFE280, and KLE were all purchased from Nanjing Cobioer. Human ovarian cancer cells OVCAR-3 and human endometrial cancer cells AN3CA were purchased from ATCC, human ovarian cancer cells SK-OV-3 were purchased from the Cell Bank of the Chinese Academy of Sciences; human endometrial cancer cells HEC-1A were purchased from Guangzhou Jennio.

Drug A was prepared by dissolving it in PBS, and drug B was prepared by dissolving it in DMSO;
CellTiter-Glo® Luminescent Cell Viability Assay was purchased from Promega, catalog number G7573;
1640 medium was purchased from Gibco, catalog number 22400-089;
McCoy 5a medium was purchased from Gibco, catalog number 16600-082;
DMEM medium was purchased from Gibco, catalog number 11995-065;
EMEM medium was purchased from ATCC, catalog number 30-2003;
FBS was purchased from Gibco, catalog number 10091-148;
Trypsin was purchased from Gibco, catalog number 25200-072;
PBS was purchased from Gibco, catalog number 10010-023.

### 1.2 Instruments:

Microplate reader (BioTek Synergy H1); pipette (Eppendorf).

### 2. Experimental methods and processing results

### 2.1 Evaluation of the inhibitory effect of the combination of drug A and drug B on the growth of human ovarian cancer, breast cancer, and endometrial cancer cells

Tumor cells in good growth condition were seeded into 96-well plates. After overnight growth onto the wall, different concentrations of the test substances were administered, with different concentrations of the two test substances arranged in a cross-combination design.

The starting concentration of test drug A was 1000 nM, which was subjected to 1:3 serial dilution to generate a total of 6 concentration levels; the starting concentration of test drug B was set at 1000 nM, which was subjected to 1:3 serial dilution to generate a total of 6 concentration levels. The specific drug treatment scheme is shown in Table 2-1. After the combined test substances had acted on each cell line for 6 days, the CTG method was used to detect the in vitro proliferation inhibitory effect of the combined use of the test drugs on tumor cells.

Table 2-1 Drug A and drug B treatment dosing scheme

| Cell line category | Cells | Drug **A** starting concentration **nM** | Drug **A** dilution ratio | Drug **B** starting concentration **nM** | Drug **B** dilution ratio |
|---|---|---|---|---|---|
| Ovarian cancer | OVCAR-3 | 1000 | 1:3 | 1000 | 1:3 |
| | SK-OV-3 | 1000 | 1:3 | 1000 | 1:3 |
| | OAW42 | 1000 | 1:3 | 1000 | 1:3 |
| | KURAMOCHI | 1000 | 1:3 | 1000 | 1:3 |
| | CAOV3 | 1000 | 1:3 | 1000 | 1:3 |
| | COV362 | 1000 | 1:3 | 1000 | 1:3 |
| | OAW28 | 1000 | 1:3 | 1000 | 1:3 |
| Breast cancer | HCC38 | 1000 | 1:3 | 1000 | 1:3 |
| | MX-1 | 1000 | 1:3 | 1000 | 1:3 |
| | CAL-148 | 1000 | 1:3 | 1000 | 1:3 |
| | CAL-51 | 1000 | 1:3 | 1000 | 1:3 |

(continued)

| Cell line category | Cells | Drug **A** starting concentration **nM** | Drug **A** dilution ratio | Drug **B** starting concentration **nM** | Drug **B** dilution ratio |
|---|---|---|---|---|---|
| Endometrial cancer | RL95-2 | 1000 | 1:3 | 1000 | 1:3 |
| | HEC-1A | 1000 | 1:3 | 1000 | 1:3 |
| | EN | 1000 | 1:3 | 1000 | 1:3 |
| | Ishikawa | 1000 | 1:3 | 1000 | 1:3 |
| | AN3CA | 1000 | 1:3 | 1000 | 1:3 |
| | EFE184 | 1000 | 1:3 | 1000 | 1:3 |
| | MFE280 | 1000 | 1:3 | 1000 | 1:3 |
| | KLE | 1000 | 1:3 | 1000 | 1:3 |

Based on the signal values measured by the microplate reader, the cell growth inhibition rate was calculated according to the following formula:
Growth inhibition rate % (growth inhibition%) = [1-(measured value-minimum value mean-background value)/(maximum value mean-background value-minimum value mean)] ×100% (measured value: reading value of compound wells; minimum value: reading value of ZPE wells (reading value of wells containing cell culture medium without cells); maximum value: reading value of HPE wells (cell reading value of the DMSO treatment group); background value: cell background measured value before addition of the compound).

The results of the cell growth inhibition rate of the combined action of drug A and drug B are shown in Tables 2-2 to 2-20. Drug A and drug B inhibited tumor-cell growth in a concentration-dependent manner over different concentration ranges. The combined action of the two drugs can significantly increase inhibition of cell growth, and the combination exhibits added or synergistic effects.

Table 2-2 Inhibitory effect of drug A and drug B in combination on the growth of OVCAR-3 cells

| **Growth inhibition rate, %** | | **Drug B concentration, nM** | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 6.53 | 10.86 | 15.23 | 14.27 | 16.02 | 28.25 |
| | **4.12** | 4.47 | 50.49 | 55.46 | 56.25 | 61.03 | 64.10 | 64.81 |
| | **12.35** | 37.52 | 80.91 | 78.20 | 78.48 | 81.37 | 83.47 | 83.94 |
| | **37.04** | 74.54 | 94.66 | 94.97 | 95.56 | 95.04 | 96.10 | 94.81 |
| | **111.11** | 98.72 | 102.67 | 101.44 | 102.04 | 100.63 | 102.43 | 101.72 |
| | **333.33** | 104.85 | 108.29 | 108.04 | 107.96 | 107.90 | 107.36 | 107.19 |
| | **1000.00** | 111.27 | 111.10 | 111.22 | 111.36 | 112.14 | 111.98 | 111.67 |

Table 2-3 Inhibitory effect of drug A and drug B in combination on the growth of SK-OV-3 cells

| **Growth inhibition rate, %** | | **Drug B concentration, nM** | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 3.96 | 2.49 | 2.34 | 6.14 | 9.22 | 8.36 |
| | **4.12** | 2.43 | 5.13 | 5.13 | 6.08 | 6.50 | 7.25 | 3.34 |
| | **12.35** | 3.74 | 5.26 | 0.42 | 2.05 | 1.70 | 1.54 | 0.32 |
| | **37.04** | -2.73 | 19.90 | 16.28 | 17.39 | 21.22 | 20.75 | 23.25 |
| | **111.11** | 31.87 | 53.09 | 52.59 | 55.45 | 56.54 | 53.75 | 55.25 |
| | **333.33** | 66.97 | 78.22 | 77.88 | 77.18 | 78.91 | 78.74 | 79.02 |
| | **1000.00** | 89.33 | 94.62 | 95.06 | 95.19 | 95.23 | 94.74 | 96.07 |

Table 2-4 Inhibitory effect of drug A and drug B in combination on the growth of OAW42 cells

| **Growth inhibition rate, %** | | **Drug B concentration, nM** | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 6.29 | 4.07 | 6.30 | 7.53 | 12.46 | 8.84 |
| | **4.12** | 2.67 | 5.27 | 7.21 | 7.82 | 10.14 | 9.74 | 12.19 |
| | **12.35** | 6.90 | 23.45 | 28.14 | 27.51 | 28.36 | 26.25 | 24.78 |
| | **37.04** | 31.03 | 55.58 | 56.15 | 56.12 | 51.20 | 53.07 | 48.47 |
| | **111.11** | 65.75 | 84.15 | 82.10 | 82.56 | 82.03 | 79.70 | 77.03 |
| | **333.33** | 83.98 | 101.04 | 102.24 | 102.29 | 105.67 | 101.58 | 103.26 |
| | **1000.00** | 108.23 | 115.71 | 117.09 | 118.70 | 118.73 | 119.30 | 117.48 |

Table 2-5 Inhibitory effect of drug A and drug B in combination on the growth of KURAMOCHI cells

| **Growth inhibition rate, %** | | **Drug B concentration, nM** | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 9.67 | 5.53 | 11.17 | 7.20 | 20.81 | 25.70 |
| | **4.12** | -6.25 | 43.68 | 42.34 | 48.32 | 52.52 | 54.28 | 63.03 |
| | **12.35** | 4.11 | 64.35 | 77.16 | 82.68 | 77.58 | 76.99 | 83.82 |
| | **37.04** | 52.01 | 96.24 | 102.14 | 102.43 | 101.91 | 96.16 | 104.89 |
| | **111.11** | 102.05 | 121.60 | 118.24 | 125.68 | 120.31 | 118.53 | 123.96 |
| | **333.33** | 128.61 | 138.84 | 136.62 | 136.65 | 138.15 | 136.86 | 139.63 |
| | **1000.00** | 138.80 | 148.58 | 148.31 | 147.06 | 147.09 | 148.64 | 149.48 |

Table 2-6 Inhibitory effect of drug A and drug B in combination on the growth of CAOV3 cells

| **Growth inhibition rate, %** | | **Drug B concentration, nM** | | | | | | |
|---|---|---|---|---|---|---|---|---|
| | | **0** | **4.12** | **12.35** | **37.04** | **111.11** | **333.33** | **1000.00** |
| **Drug A** | **0** | 0.00 | 9.96 | 12.35 | 11.36 | 18.57 | 14.64 | 33.75 |
| **concentration, nM** | **4.12** | 5.63 | 34.50 | 32.50 | 33.99 | 40.38 | 49.24 | 46.94 |
| | **12.35** | 8.12 | 74.93 | 74.96 | 74.42 | 79.83 | 82.96 | 85.82 |
| | **37.04** | 89.30 | 118.99 | 119.69 | 121.57 | 122.30 | 121.61 | 122.18 |
| | **111.11** | 126.27 | 133.05 | 132.38 | 132.89 | 133.55 | 132.25 | 132.42 |
| | **333.33** | 134.55 | 136.13 | 135.86 | 135.80 | 136.22 | 136.22 | 136.19 |
| | **1000.00** | 136.58 | 137.04 | 137.36 | 137.36 | 137.18 | 137.11 | 137.26 |

Table 2-7 Inhibitory effect of drug A and drug B in combination on the growth of COV362 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 22.59 | 22.45 | 21.18 | 25.95 | 23.74 | 30.00 |
| | **4.12** | -0.19 | 20.09 | 16.32 | 22.09 | 23.36 | 23.60 | 30.79 |
| | **12.35** | 2.89 | 22.14 | 23.68 | 28.50 | 29.98 | 28.28 | 33.70 |
| | **37.04** | 6.76 | 34.25 | 33.53 | 35.01 | 39.49 | 38.44 | 44.84 |
| | **111.11** | 35.78 | 57.87 | 59.05 | 63.45 | 64.31 | 66.08 | 66.62 |
| | **333.33** | 69.81 | 83.20 | 84.96 | 81.89 | 84.01 | 83.61 | 87.57 |
| | **1000.00** | 99.16 | 101.67 | 102.83 | 101.37 | 105.13 | 105.65 | 105.43 |

Table 2-8 Inhibitory effect of drug A and drug B in combination on the growth of OAW28 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 2.93 | 4.11 | 8.96 | -0.13 | 11.99 | 17.93 |
| | **4.12** | -2.43 | 32.89 | 22.57 | 26.76 | 30.10 | 28.62 | 33.14 |
| | **12.35** | 24.90 | 52.86 | 53.46 | 58.44 | 53.37 | 57.88 | 59.87 |
| | **37.04** | 50.88 | 71.14 | 71.14 | 70.56 | 73.43 | 75.60 | 70.83 |
| | **111.11** | 73.83 | 89.11 | 86.64 | 84.26 | 88.15 | 87.45 | 87.37 |
| | **333.33** | 92.31 | 103.19 | 103.98 | 101.10 | 102.15 | 103.51 | 104.57 |
| | **1000.00** | 112.82 | 116.49 | 116.77 | 117.86 | 118.00 | 118.25 | 118.16 |

Table 2-9 Inhibitory effect of drug A and drug B in combination on the growth of HCC38 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 2.61 | -6.97 | 1.97 | -0.02 | 4.37 | 10.72 |
| | **4.12** | -1.78 | 16.09 | 11.79 | 14.86 | 10.04 | 13.60 | 9.98 |
| | **12.35** | -3.26 | 26.69 | 22.04 | 22.63 | 23.86 | 21.42 | 17.25 |
| | **37.04** | 22.30 | 42.93 | 36.92 | 37.04 | 37.76 | 35.38 | 40.83 |
| | **111.11** | 45.08 | 61.08 | 60.55 | 55.38 | 58.40 | 59.70 | 60.08 |
| | **333.33** | 72.36 | 84.75 | 80.27 | 82.33 | 86.30 | 82.37 | 82.76 |
| | **1000.00** | 88.42 | 96.16 | 97.72 | 98.25 | 99.62 | 95.20 | 97.70 |

Table 2-10 Inhibitory effect of drug A and drug B in combination on the growth of MX-1 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 33.15 | 31.24 | 33.85 | 40.59 | 46.92 | 56.50 |
| | **4.12** | 53.44 | 94.83 | 103.19 | 104.36 | 107.33 | 106.73 | 109.32 |
| | **12.35** | 71.17 | 108.46 | 112.92 | 113.51 | 112.74 | 113.68 | 115.25 |
| | **37.04** | 90.41 | 113.86 | 116.61 | 115.29 | 115.29 | 114.65 | 117.25 |
| | **111.11** | 103.52 | 117.49 | 118.33 | 117.38 | 117.65 | 116.96 | 118.80 |
| | **333.33** | 117.01 | 119.87 | 120.45 | 119.58 | 120.51 | 119.95 | 120.38 |
| | **1000.00** | 120.33 | 121.08 | 122.14 | 121.71 | 121.71 | 122.19 | 122.81 |

Table 2-11 Inhibitory effect of drug A and drug B in combination on the growth of CAL-148 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 14.00 | 14.76 | 21.06 | 23.80 | 29.45 | 32.56 |
| | **4.12** | 8.10 | 26.91 | 29.63 | 39.60 | 43.61 | 40.90 | 49.13 |
| | **12.35** | 8.88 | 49.32 | 58.90 | 63.32 | 68.42 | 67.70 | 72.74 |
| | **37.04** | 48.36 | 91.49 | 92.10 | 91.73 | 93.09 | 92.77 | 91.18 |
| | **111.11** | 90.71 | 106.51 | 104.77 | 106.16 | 106.45 | 106.68 | 105.36 |
| | **333.33** | 107.85 | 116.32 | 113.99 | 113.69 | 115.22 | 114.61 | 114.08 |
| | **1000.00** | 120.60 | 130.13 | 129.28 | 129.29 | 129.18 | 129.30 | 129.59 |

Table 2-12 Inhibitory effect of drug A and drug B in combination on the growth of CAL-51 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 9.89 | 8.42 | 12.34 | 11.19 | 15.02 | 15.08 |
| | **4.12** | 3.61 | 6.36 | 10.54 | 13.29 | 15.37 | 15.80 | 17.81 |
| | **12.35** | 6.53 | 18.61 | 20.20 | 21.06 | 20.55 | 21.69 | 25.50 |
| | **37.04** | 20.09 | 37.04 | 38.08 | 37.78 | 37.27 | 37.74 | 42.01 |
| | **111.11** | 34.94 | 56.93 | 56.74 | 55.55 | 55.44 | 56.56 | 58.71 |
| | **333.33** | 56.13 | 77.75 | 76.74 | 77.40 | 77.28 | 79.10 | 80.17 |
| | **1000.00** | 76.82 | 94.22 | 94.89 | 95.09 | 94.74 | 95.82 | 95.21 |

Table 2-13 Inhibitory effect of drug A and drug B in combination on the growth of RL95-2 cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | -0.26 | 11.53 | 12.93 | 14.39 | 19.28 | 60.77 |
| | **4.12** | 8.02 | 28.76 | 36.81 | 38.18 | 46.11 | 43.08 | 54.85 |
| | **12.35** | 18.50 | 44.00 | 54.59 | 61.03 | 58.43 | 59.78 | 70.67 |
| | **37.04** | 24.84 | 67.69 | 76.97 | 79.31 | 81.88 | 83.76 | 85.07 |
| | **111.11** | 58.35 | 93.82 | 93.69 | 96.92 | 97.37 | 95.96 | 95.46 |
| | **333.33** | 87.69 | 100.24 | 100.06 | 101.28 | 101.07 | 100.77 | 99.55 |
| | **1000.00** | 99.39 | 102.75 | 103.22 | 103.31 | 102.84 | 103.26 | 102.21 |

Table 2-14 Inhibitory effect of drug A and drug B in combination on the growth of HEC-1A cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 3.73 | 4.25 | 5.84 | 9.01 | 7.40 | 3.87 |
| | **4.12** | 2.80 | -1.60 | 0.85 | 2.92 | 4.59 | 0.75 | 4.25 |
| | **12.35** | 2.01 | 1.66 | 2.53 | 4.92 | 5.26 | 2.41 | 5.86 |
| | **37.04** | 4.21 | 4.87 | 8.58 | 8.52 | 9.41 | 9.77 | 11.44 |
| | **111.11** | 17.09 | 38.78 | 32.98 | 33.52 | 34.77 | 34.50 | 38.11 |
| | **333.33** | 54.28 | 80.23 | 76.04 | 79.00 | 78.06 | 80.30 | 79.71 |
| | **1000.00** | 90.13 | 102.49 | 101.52 | 102.42 | 102.35 | 102.22 | 102.87 |

Table 2-15 Inhibitory effect of drug A and drug B in combination on the growth of EN cells

| Growth inhibition rate, % | | Drug B concentration, nM | | | | | | |
|---|---|---|---|---|---|---|---|---|
| | | **0** | **4.12** | **12.35** | **37.04** | **111.11** | **333.33** | **1000.00** |
| **Drug A concentration, nM** | **0** | 0.00 | 31.13 | 22.59 | 24.15 | 27.18 | 24.54 | 36.45 |
| | **4.12** | -3.99 | 29.49 | 26.82 | 31.80 | 31.23 | 30.92 | 38.14 |
| | **12.35** | -0.33 | 30.42 | 30.75 | 29.57 | 33.77 | 35.19 | 39.21 |
| | **37.04** | 17.51 | 41.04 | 41.43 | 38.00 | 40.44 | 46.73 | 42.07 |
| | **111.11** | 28.07 | 47.75 | 49.21 | 45.18 | 49.47 | 48.52 | 52.76 |
| | **333.33** | 47.02 | 54.54 | 54.53 | 53.91 | 53.78 | 55.55 | 55.65 |
| | **1000.00** | 54.99 | 53.06 | 53.50 | 56.32 | 51.55 | 50.66 | 53.70 |

Table 2-16 Inhibitory effect of drug A and drug B in combination on the growth of Ishikawa cells

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| Drug A concentration, nM | 0 | 0.00 | 4.28 | -0.41 | 6.94 | 8.97 | 16.13 | 10.87 |
| | 4.12 | -6.87 | -8.94 | 2.81 | 13.68 | 14.27 | 20.04 | 23.56 |
| | 12.35 | -5.24 | 17.54 | 12.95 | 21.30 | 25.52 | 13.69 | 25.00 |
| | 37.04 | 2.50 | 28.17 | 33.73 | 45.89 | 46.02 | 43.78 | 47.76 |
| | 111.11 | 28.73 | 67.44 | 67.45 | 71.40 | 70.31 | 70.12 | 63.15 |
| | 333.33 | 61.51 | 85.19 | 81.19 | 83.42 | 82.30 | 81.37 | 77.57 |
| | 1000.00 | 86.29 | 87.82 | 87.21 | 87.69 | 88.13 | 86.88 | 86.71 |

Table 2-17 Inhibitory effect of drug A and drug B in combination on the growth of AN3CA cells

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| Drug A concentration, nM | 0 | 0.00 | 9.56 | 15.12 | 15.88 | 22.44 | 21.37 | 25.35 |
| | 4.12 | 0.46 | 10.00 | 21.84 | 26.07 | 30.06 | 31.51 | 33.83 |
| | 12.35 | 5.95 | 25.66 | 33.52 | 42.24 | 41.63 | 43.97 | 45.69 |
| | 37.04 | 26.35 | 57.48 | 54.54 | 70.35 | 58.79 | 59.25 | 51.96 |
| | 111.11 | 49.20 | 77.91 | 81.08 | 85.82 | 75.90 | 82.37 | 83.52 |
| | 333.33 | 72.86 | 83.61 | 85.13 | 83.85 | 85.03 | 82.30 | 83.41 |
| | 1000.00 | 84.69 | 80.00 | 85.63 | 86.81 | 83.65 | 85.13 | 87.60 |

Table 2-18 Inhibitory effect of drug A and drug B in combination on the growth of EFE184 cells

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| Drug A concentration, nM | 0 | 0.00 | 2.97 | -0.06 | -3.28 | -6.21 | -1.81 | 0.06 |
| | 4.12 | 2.01 | 6.83 | 2.46 | 1.81 | -0.53 | -0.48 | 2.70 |
| | 12.35 | 0.43 | 4.91 | 2.08 | 0.58 | -1.16 | -1.44 | -0.27 |
| | 37.04 | 0.63 | 4.34 | 1.66 | -0.55 | -1.76 | -0.45 | 1.56 |
| | 111.11 | -0.67 | 7.81 | 8.11 | 8.20 | 5.40 | 3.80 | 4.45 |
| | 333.33 | 8.39 | 39.10 | 42.11 | 43.40 | 37.82 | 33.87 | 33.47 |
| | 1000.00 | 58.11 | 70.45 | 71.38 | 72.27 | 70.93 | 68.99 | 69.49 |

Table 2-19 Inhibitory effect of drug A and drug B in combination on the growth of MFE280 cells

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| Drug A concentration, nM | 0 | 0.00 | -7.63 | 0.40 | 10.75 | 29.83 | 73.14 | 108.23 |
| | 4.12 | 19.72 | 47.56 | 35.55 | 34.19 | 30.23 | 35.54 | 36.92 |
| | 12.35 | 16.77 | 37.78 | 30.20 | 30.60 | 29.19 | 31.05 | 33.18 |
| | 37.04 | 19.65 | 46.74 | 39.12 | 38.87 | 36.56 | 37.39 | 41.10 |
| | 111.11 | 17.19 | 66.28 | 65.60 | 63.78 | 62.51 | 60.63 | 60.51 |
| | 333.33 | 20.32 | 94.29 | 93.32 | 92.46 | 90.33 | 90.52 | 85.54 |

(continued)

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| | 1000.00 | 30.22 | 116.05 | 115.06 | 116.16 | 115.38 | 114.97 | 112.56 |

Table 2-20 Inhibitory effect of drug A and drug B in combination on the growth of KLE cells

| Growth inhibition rate, % | | Drug B concentration, nM | | | | | | |
|---|---|---|---|---|---|---|---|---|
| | | 0 | 4.12 | 12.35 | 37.04 | 111.11 | 333.33 | 1000.00 |
| Drug A concentration, nM | 0 | 0.00 | -4.53 | -4.09 | -0.35 | -0.57 | 1.33 | 9.21 |
| | 4.12 | -8.41 | -0.52 | -4.38 | -2.55 | -8.10 | -3.69 | -3.32 |
| | 12.35 | -5.41 | 5.61 | -1.20 | 0.78 | -1.36 | -0.86 | 2.91 |
| | 37.04 | -2.11 | 21.18 | 16.59 | 20.43 | 21.89 | 17.21 | 23.43 |
| | 111.11 | 23.79 | 50.45 | 49.17 | 47.60 | 48.95 | 48.41 | 48.03 |
| | 333.33 | 55.62 | 75.67 | 75.86 | 74.19 | 73.98 | 70.36 | 74.06 |
| | 1000.00 | 77.04 | 92.11 | 89.91 | 91.72 | 91.26 | 92.11 | 91.62 |

**2.2 Evaluation of the combined effect of drug A in combination with drug B on inhibition of tumor cell growth**

Based on the experimental results of Experiment 2.1, the cell growth inhibition rate was converted into cell survival rate, the data of the tumor cell growth inhibition experiment were analyzed using Combenefit software (Di Veroli et al., 2016, Bioinformatics 32(18): 2866-2868), the Bliss model was used to evaluate synergistic/antagonistic effects, and the combined index (combined index, CI) of the two drugs was analyzed and calculated. Wherein, CI > 10 indicates a pharmacodynamic synergistic effect, -10 < CI < 10 indicates a pharmacodynamic additive effect, and CI < -10 indicates a pharmacodynamic antagonistic effect.

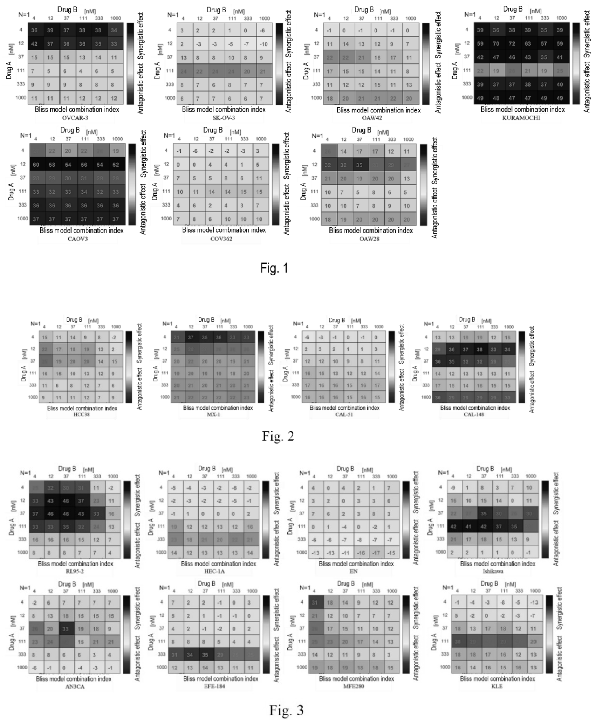

Figure 1, Figure 2, and Figure 3 show that, in the vast majority of cell lines, the combined index of the two drugs has CI > 10, indicating that the two drugs have a pharmacodynamic synergistic effect in the vast majority of cell lines; in addition, under different concentration combinations of the two drugs, the vast majority of the CI values are > -10 or > 10, indicating that under different concentration combinations of the two drugs, there is a pharmacodynamic additive effect or a pharmacodynamic synergistic effect.

**3. Experimental conclusion:**

The combined use of drug A and drug B, as compared with single-drug use, can improve the inhibitory effect on the growth of tumor cells, and the combined use of drug A and drug B has a pharmacodynamic synergistic effect and a pharmacodynamic additive effect.

**Example 3.** Evaluation of the in vivo inhibitory effect of the combined use of drug A and drug B on MX-1 human breast cancer cell mouse xenografts

**1. Experimental materials**

Drug A was prepared by dilution with normal saline, and drug B was prepared using 0.5% HPMC;
Human breast cancer MX-1 cells were cultured as a monolayer in vitro under culture conditions of DMEM/F12 medium supplemented with 10% fetal bovine serum, 100 U/mL penicillin, and 100 μg/mL streptomycin, and cultured in a cell incubator at 37°C and 5% $CO_2$. Routine digestion treatment and passaging were carried out twice a week using trypsin-EDTA. When the cell confluence was 80%-90% and the number of cells reached the requirement, the cells were collected, counted, and inoculated.

Balb/c nude mice, female, body weight 17-25 g, were purchased from Shanghai Bi Kai Ke Yi Biotechnology Co., Ltd.

**2. Experimental method**

MX-1 cells were resuspended in PBS, the density after resuspension was $100 \times 10^6$/mL, the resuspended cells were mixed with an equal volume of Matrigel, inoculated subcutaneously into the right dorsal side of each mouse, 0.1 mL was inoculated into each mouse ($5 \times 10^6$ cells/animal), and when the average tumor volume grew to 100-150 $mm^3$, grouping was carried out (D1), and drug administration was started on the day of grouping. The mice were administered by gavage (*p.o.*) once daily; the mice were administered by tail vein injection (*i.v.*) as a single administration; the administration volume was 10 mL/kg; the vehicle group was given the same volume of "vehicle"; the specific administration doses and administration regimens are shown in Table 3-1. Tumor volume was measured, mouse body weight was weighed, and data were recorded.

The experimental indicator was to examine the effect of the drug on tumor growth, and the specific indicator was the tumor growth inhibition rate TGI (%).

The tumor diameter was measured twice a week with a vernier caliper, and the calculation formula for tumor volume (V) was:$V = 1/2 \times a \times b^2$ where a, b respectively indicate length and width.

Calculation of TGI (%): When there was no tumor regression, TGI (%) = [1 - (average tumor volume of a certain treatment group at the end of administration - average tumor volume of that treatment group at grouping)/(average tumor volume of the vehicle control group at the end of treatment - average tumor volume of the vehicle control group at grouping)] × 100%. When there was tumor regression, TGI (%) = [1 - (average tumor volume of a certain treatment group at the end of administration - average tumor volume of that treatment group at grouping)/average tumor volume of that treatment group at grouping] × 100%.

At the end of the experiment, upon reaching the experimental endpoint, or when the average tumor volume reached 2000 $mm^3$, the animals were euthanized under $CO_2$ anesthesia, and then dissected for tumor collection and photographing.

The experimental data were analyzed and plotted using GraphPad Prism 9.4. Based on the tumor volume data of each group at different time points, Dunnett's multiple comparisons test in Two-way ANOVA was used for statistical analysis to evaluate differences among groups. A t-test was used to analyze the difference in tumor volume between two groups, and $p < 0.05$ was defined as indicating that the difference was statistically significant.

Table 3-1. Dosing regimen of drug A in combination with drug B in the MX-1 model

| Group | Grouping | Dose administered mg/kg | Administration volume mL/kg | Administration schedule | Route of administration |
|---|---|---|---|---|---|
| 1 | Vehicle | - | 10 | QD*28 times | *p.o.* |
| 2 | Drug B | 0.3 | 10 | QD*28 times | *p.o.* |
| 3 | Drug A | 1 | 10 | Single administration | *i.v.* |
| 4 | Drug A | 2 | 10 | Single administration | *i.v.* |
| 5 | Drug B + Drug A | 0.3 + 1 | 10 + 10 | QD*28 times + single administration | *p.o.* + *i.v.* |
| 6 | Drug B + Drug A | 0.3 + 2 | 10 + 10 | QD*28 times + single administration | *p.o.* + *i.v.* |

**3. Experimental results**

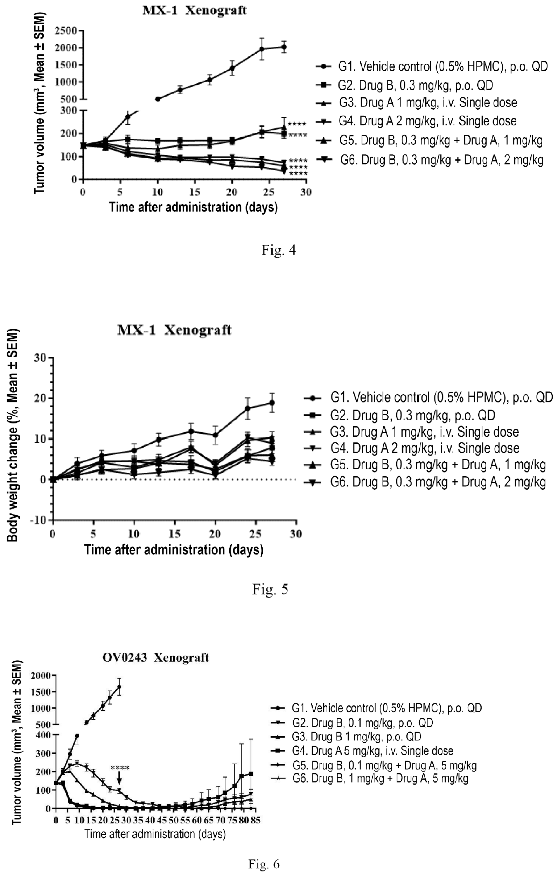

The growth inhibitory effect of drug A in combination with drug B on the MX-1 model is shown in Table 3-2 and Figure 4, and the body weight changes of animals in each group in the MX-1 model are shown in Figure 5. During the experiment, the average body weight of the animals in each group was relatively stable. In addition, no treatment-related death or other abnormal symptoms occurred in the mice, indicating that the tumor-bearing mice could well tolerate single-drug treatment with test drug A, single-drug treatment with drug B, and combined treatment with drugs A and B.

Table 3-2. Growth inhibitory effect of drug A in combination with drug B on the MX-1 model

| **Group** | **Average tumor volume ($mm^3$) ± SEM** | | **TGI (%)** | ***p* value** | **Partial regression** |
|---|---|---|---|---|---|
| | **D0** | **D28** | **D28** | **D28** | **D28** |
| Vehicle | 147±7 | 2025±170 | - | - | 0/8 |

(continued)

| Group | Average tumor volume ($mm^3$) ± SEM | | TGI (%) | *p* value | Partial regression |
|---|---|---|---|---|---|
| | D0 | D28 | D28 | D28 | D28 |
| Drug B_0.3 mg/kg | 148±7 | 199±20 | 97.25 | <0.0001 | 2/8 |
| Drug A_1 mg/kg | 147±7 | 226±42 | 95.80 | <0.0001 | 2/8 |
| Drug A_2 mg/kg | 148±8 | 74±11 | 149.92 | <0.0001 | 7/8 |
| Drug B_0.3 mg/kg + Drug A_1 mg/kg | 147±7 | 59±7 | 159.86 | <0.0001 | 8/8 |
| Drug B_0.3 mg/kg + Drug A_2 mg/kg | 147±8 | 37±4 | 175.03 | <0.0001 | 8/8 |

*p* value D28: the value obtained by performing Dunnett analysis using Two-way ANOVA based on the tumor volume of each animal in different groups with the vehicle group as the control; in addition, t-test was used for statistical analysis, and the drug B_0.3 mg/kg combined with drug A_1 mg/kg group was respectively compared with the drug B _0.3 mg/kg group and the drug A _1 mg/kg group, with p values of < 0.0001 and 0.0015, respectively. The combined drug B _0.3 mg/kg and drug A_2 mg/kg group was respectively compared with the drug B _0.3 mg/kg group and the drug A _2 mg/kg group, with p values of < 0.0001 and 0.2139, respectively.

**4. Experimental conclusion:**

In the MX-1 human breast cancer xenograft mouse model, the tumor-bearing mice tolerated both drug A alone and in combination with drug B, the antitumor efficacy of the combined drug B_0.3 mg/kg + drug A_1 mg/kg treatment group was significantly greater than that of each monotherapy group, and the antitumor efficacy of the combined drug B_0.3 mg/kg and drug A_2 mg/kg treatment group was significantly superior to the drug B_0.3 mg/kg monotherapy treatment group and slightly superior to the drug A_2 mg/kg monotherapy treatment group.

**Example 4.** Evaluation of the in vivo inhibitory effect of the combined use of drug A and drug B on human ovarian cancer OV0243 in a mouse subcutaneous xenograft tumor model

**1. Experimental materials**

Drug A was prepared by dilution with normal saline, and drug B was prepared using 0.5% HPMC;
OV0243 (mild cachexia), human ovarian cancer tumor tissue, was provided by Crown Bioscience (Beijing) Inc.
BALB/c nude mice, female, body weight 16-22 g, were purchased from Jiangsu Jicui Yaokang Biotechnology Co., Ltd.

**2. Experimental method**

OV0243 human ovarian cancer tissue was uniformly cut into tumor pieces with a diameter of 2-3 mm and inoculated subcutaneously at the right front scapular region of BALB/c nude mice, and tumor growth was regularly observed. When the average tumor volume reached 100-150 $mm^3$, random grouping was performed based on the tumor size and mouse body weight in accordance with Table 4-1. The tumor diameter was measured twice a week with a vernier caliper, and the calculation formula for tumor volume (V) was: $V = 1/2 \times a \times b^2$ where a, b respectively indicate length and width.

The experimental indicator was to examine the effect of the drug on tumor growth, and the specific indicator was the tumor growth inhibition rate TGI (%).

Calculation of TGI (%): When there was no tumor regression, TGI (%) = [1 - (average tumor volume of a certain treatment group at the end of administration - average tumor volume of that treatment group at grouping)/(average tumor volume of the vehicle control group at the end of treatment - average tumor volume of the vehicle control group at grouping)] × 100%. When there was tumor regression, TGI (%) = [1 - (average tumor volume of a certain treatment group at the end of administration - average tumor volume of that treatment group at grouping)/average tumor volume of that treatment group at grouping] × 100%.

At the end of the experiment, upon reaching the experimental endpoint, or when the average tumor volume reached 2000 $mm^3$, the animals were euthanized under $CO_2$ anesthesia, and then dissected for tumor collection and photographing.

The experimental data were analyzed and plotted using GraphPad Prism 9.4. Based on the tumor volume data of

each group at different time points, Dunnett's multiple comparisons test in Two-way ANOVA was used for statistical analysis to evaluate differences among groups. A t-test was used to analyze the difference in tumor volume between two groups, and $p < 0.05$ was defined as indicating that the difference was statistically significant.

The invivoSyn method was used to analyze the synergistic effect of the combination treatment. First, based on the exponential growth model, the tumor growth rate of each mouse (eGR, exponential growth rate) and the average tumor growth rate of each group (eGRg) were calculated. Then, the relative survival rate of the treatment group and the control group was calculated by using the conditional probability formula $Pr(T|C)=S_T/S_C$, wherein $S_A$, $S_B$, and $S_{AB}$ are respectively defined as the relative survival rates of single-drug group A, single-drug group B, and the combination administration group at time point t.

$$S_A = \frac{\exp(eGR_A * t)}{\exp(eGR_C * t)}$$

$$S_B = \frac{\exp(eGR_B * t)}{\exp(eGR_C * t)}$$

$$S_{AB} = \frac{\exp(eGR_{AB} * t)}{\exp(eGR_C * t)}$$

Based on the highest single-drug effect model (HSA, Highest Single Agent), the expected survival rate $E(S_{AB})$ of the combination administration group was calculated. For the HSA model, $E(S_{AB}) = \min(S_A, S_B)$. CI (Combination Index) is defined as the ratio of the observed survival rate $S_{AB}$ of the combination administration group to $E(S_{AB})$, while the synergistic effect score (SS, Synergy Score) is defined as the percentage difference between $E(S_{AB})$ and $S_{AB}$. When the CI value is less than 1 or the SS value is greater than 0, the combination is considered to have a synergistic effect. *p* values and confidence intervals were calculated using the stratified bootstrap method (Stratified bootstrap).

Table 4-1. Dosing regimen of drug A in combination with drug B in the OV0243 model

| Group | Grouping | Dose administered mg/kg | Administration volume mL/kg | Administration schedule | Route of administration |
|---|---|---|---|---|---|
| 1 | Vehicle | - | 10 | QD*28 times | *p.o.* |
| 2 | Drug B | 0.1 | 10 | QD*28 times | *p.o.* |
| 3 | Drug B | 1 | 10 | QD*28 times | *p.o.* |
| 4 | Drug A | 5 | 10 | Single administration | *i.v.* |
| 5 | Drug B + Drug A | 0.1 + 5 | 10 + 10 | QD*28 times + single administration | *p.o.* + *i.v.* |
| 6 | Drug B + Drug A | 1 + 5 | 10 + 10 | QD*28 times + single administration | *p.o.* + *i.v.* |

### 3. Experimental results

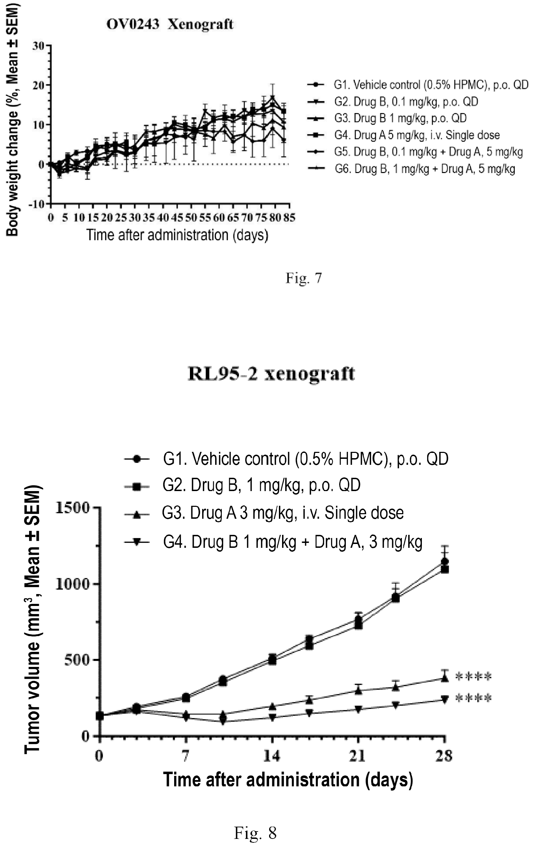

The growth inhibitory effect of drug A in combination with drug B on the OV0243 model is shown in Table 4-2 and Figure 6, and the synergistic effect analysis of drug A in combination with drug B is shown in Table 4-3. The body weight changes of animals in each group in the OV0243 model are shown in Figure 7.

On day 27 after administration, in the drug B_0.1 mg/kg treatment group, 5 animals (5/6) showed partial tumor regression, and in the drug B_1 mg/kg treatment group, 3 animals (3/6) showed partial tumor regression and 3 animals (3/6) showed complete tumor regression. In the drug A_5 mg/kg treatment group, 5 animals (5/6) showed complete tumor regression, and 1 animal (1/6) showed partial tumor regression. In the combined drug B_0.1 mg/kg and drug A_5 mg/kg treatment group and the combined drug B_1 mg/kg and drug A_5 mg/kg treatment group, all animals showed complete tumor regression.

Statistics based on the D0-D27 data showed that, compared with the vehicle group, the average tumor volume of each treatment group decreased to different extents, and all had statistically significant differences ($p < 0.0001$). In addition, based on the tumor volume on day 27 after administration, the tumor growth inhibitory effects among the

treatment groups were compared, and statistics showed that the tumor growth inhibitory effect of the combined drug B_0.1 mg/kg and drug A_5 mg/kg treatment group was significantly superior to that of the drug B_0.1 mg/kg treatment group ($p < 0.0001$), and slightly superior to that of the drug A_5 mg/kg treatment group ($p = 0.3409$). The combined drug B_1 mg/kg and drug A_5 mg/kg treatment group was slightly superior to both the drug B_1 mg/kg treatment group and the drug A_5 mg/kg treatment group ($p = 0.0950$, $p = 0.3409$).

Based on the tumor volume on day 83 after administration (that is, day 56 after drug withdrawal), statistics showed that the tumor growth inhibitory effect of the combined drug B_0.1 mg/kg and drug A_5 mg/kg treatment group was significantly superior to that of the drug B_0.1 mg/kg treatment group ($p = 0.0101$), and slightly superior to that of the drug A_5 mg/kg treatment group ($p = 0.3409$). The combined drug B_1 mg/kg and drug A_5 mg/kg treatment group was slightly superior to the drug B_1 mg/kg treatment group and the drug A_5 mg/kg treatment group ($p = 0.0603$, $p = 0.3409$).

When drug A_5 mg/kg was used in combination with drug B_0.1 mg/kg and drug B_1 mg/kg, respectively, the CI values based on the HSA model were both less than 1 (0.724 and 0.741, respectively), the Synergy Scores (SS) were both greater than 0 (0.503 and 0.471, respectively), and the *p* values were both less than 0.05. Therefore, both combination regimens had significant synergistic effects, and the pharmacological effect of the combination treatment groups was significantly higher than the pharmacological effect of single-drug treatment groups.

During the experiment, the average body weight of the animals in the vehicle group was maintained well, and on day 27, the average body weight change rate of the animals in this group was 5.70%. The average body weight change rates of the drug B_0.1 mg/kg treatment group, the drug B_1 mg/kg treatment group, the drug A_5 mg/kg treatment group, the combined drug B_0.1 mg/kg and drug A_5 mg/kg treatment group, and the combined drug B_1 mg/kg and drug A_5 mg/kg treatment group were 2.18%, 3.75%, 4.59%, 2.61%, and 2.85%, respectively. The OV0243 model exhibited mild cachexia. During the administration period, in the drug B_0.1 mg/kg treatment group, the body weight of 1 animal decreased by 17.39% on day 23, administration was stopped (Day23-27) and nutritional gel was supplemented, the mouse body weight never recovered, and by day 51 the body weight decrease was greater than 20%, so euthanasia was carried out in accordance with animal ethics (it cannot be excluded that individual variation resulted in poor drug tolerance in this mouse or that the mouse had an underlying disease). Apart from this, throughout the entire experiment there was no discontinuation of administration caused by body weight decrease in other animals, and the animals also had no disease onset or death, indicating that the tumor-bearing mice could well tolerate single-drug treatment with test drug A, single-drug treatment with drug B, and combination treatment with drugs A and B.

Table 4-2. Growth inhibitory effect of drug A in combination with drug B on the OV0243 model

| Group | Average tumor volume ($mm^3$) ± SEM | | | | TGI (%) | *p* value |
|---|---|---|---|---|---|---|
| | D0 | Day 27 | Day 55 | Day 83 | D27 | D27 |
| **Vehicle** | 137±2 | 1653±261 | - | - | - | - |
| **Drug B_0.1 mg/kg** | 137±2 | 96±14 | 3±3 | 78±27 | 129.84 | < 0.0001 |
| **Drug B_1 mg/kg** | 138±2 | 11±6 | 0±0 | 50±23 | 191.94 | < 0.0001 |
| **Drug A_5 mg/kg** | 137±2 | 2±2 | 20±20 | 188±188 | 198.26 | < 0.0001 |
| **Drug B_0.1 mg/kg + Drug A_5 mg/kg** | 138±3 | 0±0 | 0±0 | 0±0 | 200.00 | < 0.0001 |
| **Drug B_1 mg/kg + Drug A_5 mg/kg** | 138±2 | 0±0 | 0±0 | 0±0 | 200.00 | < 0.0001 |

*p* value D27: the values obtained by performing Dunnett analysis using Two-way ANOVA based on the tumor volume of each animal in different groups with the vehicle group as the control; in addition, based on the tumor volume on day 27 after administration, t-test was used for statistical analysis, and for comparison of the combined drug B_0.1 mg/kg and drug A_5 mg/kg group with the drug B_0.1 mg/kg group and the drug A_5 mg/kg group, the *p* values were < 0.0001 and 0.3409, respectively. In the comparison of the combined drug B_1 mg/kg and drug A_5 mg/kg group with the drug B_1 mg/kg group and the drug A_5 mg/kg group, the *p* values were 0.0950 and 0.3409, respectively, and there was no statistically significant difference. Based on the tumor volume on day 83 after administration, t-test was used for statistical analysis, and for comparison of the drug B_0.1 mg/kg combined with drug A_5 mg/kg group with the drug B_0.1 mg/kg group and the drug A_5 mg/kg group, the *p* values were 0.0101 and 0.3409, respectively. In the comparison of the combined drug B_1 mg/kg and drug A_5 mg/kg group with the drug B_1 mg/kg group and the drug A_5 mg/kg group, the *p* values were 0.0603 and 0.3409, respectively.

Table 4-3. Synergistic effect analysis of treatments with drug A alone and in combination with drug B based on the HSA model

| Group | CI | | | SS | | | Conclusion |
|---|---|---|---|---|---|---|---|
| | CI value | 95% confidence interval | *p* value | SS value | 95% confidence interval | *p* value | |
| Drug B_0.1 mg/kg + Drug A_5 mg/kg | 0.724 | (0.268.0.971) | 0.007 | 0.503 | (0.038.3.248) | 0.007 | Synergy |
| Drug B_1 mg/kg + Drug A_5 mg/kg | 0.741 | (0.407.0.986) | 0.021 | 0.471 | (0.014.2.423) | 0.021 | Synergy |

**4. Experimental conclusion:**

In the OV0243 human ovarian cancer xenograft mouse model, the tumor-bearing mice could well tolerate single-drug treatment with test drug A, single-drug treatment with drug B, and combined treatment with drugs A and B. Drug A monotherapy and drug B monotherapy could both significantly inhibit the growth of xenograft tumors in nude mice, and the combined drug A and drug B_0.1 mg/kg and the combined drug A and drug B_1 mg/kg likewise showed significant tumor growth inhibitory effects, slightly superior to drug A and drug B monotherapy, and the combination treatment groups both had significant synergistic effects.

**Example 5.** Evaluation of the in vivo inhibitory effect of the combined use of drug A and drug B on RL95-2 endometrial cancer cell mouse xenografts

**1. Experimental materials**

Drug A was prepared by dilution with normal saline, and drug B was prepared using 0.5% HPMC;
Human endometrial cancer cells RL95-2 were cultured in vitro in a cell incubator at 37°C and 5% $CO_2$. Routine digestion treatment and passaging were carried out twice a week using trypsin-EDTA. When the cell confluence was 80%-90% and the number of cells reached the requirement, the cells were collected, counted, and inoculated.

**2. Experimental method**

RL95-2 cells were resuspended in PBS, the density after resuspension was $100 \times 10^6$/mL, the resuspended cells were mixed with an equal volume of Matrigel, inoculated subcutaneously into the right dorsal side of each mouse, 0.1 mL was inoculated into each mouse ($5 \times 10^6$ cells/animal), and when the average tumor volume grew to 100-150 $mm^3$, grouping was carried out (D1), and administration was started on the day of grouping. The mice were administered by gavage (*p.o.*) once daily; the mice were administered by tail vein injection (*i.v.*) as a single administration; the administration volume was 10 mL/kg; the vehicle group was given the same volume of "vehicle"; the specific administration doses and administration regimens are shown in Table 5-1. Tumor volume was measured, mouse body weight was weighed, and data were recorded.

The experimental indicator was to examine the effect of the drug on tumor growth, and the specific indicator was the tumor growth inhibition rate TGI (%).

The tumor diameter was measured twice a week with a vernier caliper, and the calculation formula for tumor volume (V) was: $V = 1/2 \times a \times b^2$ where a, b respectively indicate length and width.

Calculation of TGI (%): When there was no tumor regression, TGI (%) = [1 - (average tumor volume of a certain treatment group at the end of administration - average tumor volume of that treatment group at grouping)/(average tumor volume of the vehicle control group at the end of treatment - average tumor volume of the vehicle control group at grouping)] × 100%. When there was tumor regression, TGI (%) = [1 - (average tumor volume of a certain treatment group at the end of administration - average tumor volume of that treatment group at grouping)/average tumor volume of that treatment group at grouping] × 100%.

At the end of the experiment, upon reaching the experimental endpoint, or when the average tumor volume reached 2000 $mm^3$, the animals were euthanized under $CO_2$ anesthesia, and then dissected for tumor collection and photographing.

The experimental data were analyzed and plotted using GraphPad Prism 9.4. Based on the tumor volume data of each group at different time points, Dunnett's multiple comparisons test in Two-way ANOVA was used for statistical analysis to evaluate differences among groups. A t-test was used to analyze the difference in tumor volume between two

groups, and $p < 0.05$ was defined as indicating that the difference was statistically significant.

Table 5-1. Dosing regimen of drug A in combination with drug B in the RL95-2 model

| Group | Grouping | Dose administered mg/kg | Administration volume mL/kg | Administration schedule | Route of administration |
|---|---|---|---|---|---|
| 1 | Vehicle | - | 10 | QD*28 times | *p.o.* |
| 2 | Drug B | 1 | 10 | QD*28 times | *p.o.* |
| 3 | Drug A | 3 | 10 | Single administration | *i.v.* |
| 4 | Drug B + Drug A | 1+3 | 10 + 10 | QD*28 times + single administration | *p.o.* + *i.v.* |

**3. Experimental results**

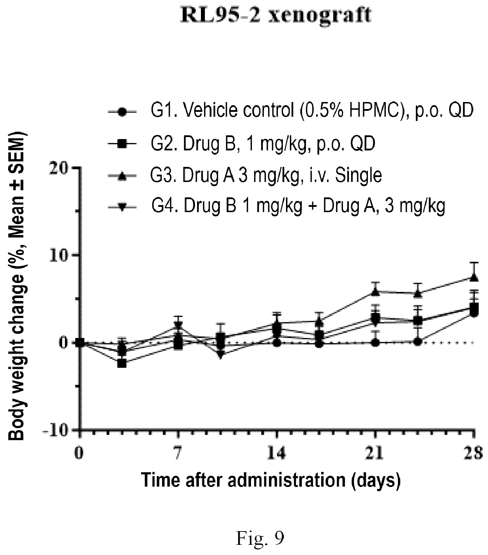

The growth inhibitory effect of drug A in combination with drug B on the RL95-2 model is shown in Table 5-2 and Figure 8, and the body weight changes of animals in each group in the RL95-2 model are shown in Figure 9. In the RL95-2 human endometrial cancer xenograft mouse model, the tumor-bearing mice could well tolerate single-drug treatment with test drug A, single-drug treatment with drug B, and combined treatment with drugs A and B. Drug A alone could significantly inhibit the growth of xenograft tumors in nude mice, whereas drug B alone had no obvious inhibitory effect on tumor growth; the combined use of drug A and drug B showed a significant tumor growth inhibitory effect and was significantly superior to single-drug treatments with either drug A or drug B in efficacy.

Table 5-2. Growth inhibitory effect of drug A in combination with drug B on the RL95-2 model

| Group | Average tumor volume ($mm^3$) + SEM | | T/C (%) | TGI (%) | *p* value |
|---|---|---|---|---|---|
| | D0 | D28 | D28 | D28 | D28 |
| Vehicle | 134±8 | 1148±100 | - | - | - |
| Drug B_1 mg/kg | 134+7 | 1097+109 | 95.50 | 5.08 | 0.9793 |
| Drug A_ 3 mg/kg | 134±8 | 380+53 | 33.14 | 75.72 | <0.0001 |
| Drug B_1 mg/kg + Drug A_ 3 mg/kg | 134±8 | 238+24 | 20.72 | 89.79 | <0.0001 |

*p* value D28: the value obtained by performing Dunnett analysis using Two-way ANOVA based on the tumor volume of each animal in different groups with the vehicle group as the control; in addition, t-test was used for statistical analysis, and the drug B_1 mg/kg combined with drug A_ 3 mg/kg group was respectively compared with the B_1 mg/kg group and the A_ 3 mg/kg group, and the p values were < 0.0001 and 0.0270, respectively.

**Example 6.** In vivo pharmacodynamic study of drug A monotherapy or combination therapy in a mouse subcutaneous xenograft model of the human ovarian cancer cell line OVCAR3

1. Test drugs

Drug A: prepared by the method in Example 1, with normal saline used for drug preparation.
Drug B: 1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was prepared by the method disclosed in WO2022223025, and was used as drug B.
Drug C: bevacizumab for injection, provided by Suzhou Suncadia Biopharmaceuticals Co., Ltd., with normal saline used for drug preparation.

2. Experimental instruments and reagents

2.1 Instruments
Carbon dioxide incubator (HERAcell-240i, Thermo Fisher Scientific); precision balance (SECURA225D-1CN,

Sartorius Group, Germany); general balance (HZ2002A, Changzhou Tianzhiping Instrument Equipment Co., Ltd.; biosafety cabinet (BSC1300-II-A2, Shandong Xinhua Medical Devices Co., Ltd.); digital caliper ((0-150) mm/0.01 mm, Mitutoyo, Japan); pipettes (20-200 μL; 100-1000 μL, Eppendorf)

2.2 Reagents

RPMI 1640 was purchased from Gibco, catalog number 22400-071; FBS was purchased from Gibco, catalog number 10091148; PBS was purchased from Gibco, catalog number 10010023; trypsin was purchased from Gibco, catalog number 25200056; Matrigel was purchased from Corning, catalog number 354234.

3. Experimental procedures and data processing

3.1 Animals

NOD SCID mice, 6-8 weeks old, female, purchased from Jiangsu Jicui Yaokang Biotechnology Co., Ltd.

3.2 Cell culture and preparation of cell suspension

a, One strain of OVCAR3 cells was taken out from the cell bank, the cells were resuscitated with RPMI 1640 medium, and the resuscitated cells were placed in a cell culture flask (with the cell type, date, name of the culturing person, etc. marked on the flask wall) and cultured in a $CO_2$ incubator (the incubator temperature was 37°C, and the $CO_2$ concentration was 5%).

b, The cells were passaged once a week, and after passaging, the culture of cells was continued in a $CO_2$ incubator. This process was repeated until the number of cells met the requirements for in vivo pharmacodynamic study.

c, The cultured cells were collected and counted with a fully automatic cell counter, the cells were resuspended with PBS according to the counting result to prepare a cell suspension (density $10 \times 10^7$/mL), an equal volume of Matrigel was added to the cell suspension and mixed uniformly, and the mixture was placed in an ice box for use later. 3.3 Cell inoculation

a, Before inoculation, the nude mice were marked with disposable universal ear tags for rats and mice;

b, At the time of inoculation, the cell suspension was mixed uniformly, 0.2-1 mL of the cell suspension was drawn with a 1 mL syringe and air bubbles were expelled, and then the syringe was placed on an ice pack for use later;

c, The NOD SCID mouse was properly restrained with the left hand, the position on the right back of the nude mouse near the right shoulder (the inoculation site) was disinfected with a 75% alcohol cotton ball, and inoculation was started after 30 seconds;

d, The experimental NOD SCID mice were inoculated in sequence (each mouse was inoculated with 0.1 mL of the cell suspension).

3.4 Tumor measurement, grouping, and administration in tumor-bearing mice

a, According to the tumor growth condition, tumor measurement was carried out on days 20-30 after the inoculation, and the tumor size was calculated;

Tumor volume calculation: tumor volume ($mm^3$) = length (mm) × width (mm) × width (mm)/2

b, According to the body weight and tumor size of the tumor-bearing mice, grouping was carried out by a random grouping method;

c, According to the grouping results, administration of the test drugs was initiated, and the specific doses and regimens are shown in Table 6-1.

**Table 6-1. Administration and grouping**

| Group | Compound | Administration volume (μL/g) [a] | Route of administration | Administration frequency |
|---|---|---|---|---|
| **1** | Vehicle | 10 | *i.v.* | Single dose |
| **2** | Drug A | 10 | *i.v.* | Single dose |
| **3** | Drug B | 10 | *p.o.* | QD × 3w |

(continued)

| Group | Compound | Administration volume (μL/g) [a] | Route of administration | Administration frequency |
|---|---|---|---|---|
| **4** | Drug C | 10 | *i.v.* | BIW × 3w |
| **5** | Drug A + Drug B | 10 + 10 | *i.v.* + *p.o.* | Single dose + QD × 3w |
| **6** | Drug A+ Drug C | 10 + 10 | *i.v.* + *i.v.* | Single dose + BIW × 3w |
| **7** | Drug B+ Drug C | 10 + 10 | *p.o.*+ *i.v.* | QD × 3w + BIW × 3w |
| **8** | Drug A+ Drug B+ Drug C | 10 + 10+ 10 | *i.v.* + *i.v.* + *i.v.* | Single dose +QD × 3w + BIW × 3w |

a. Administration volume: administration was carried out at 10 μL/g based on mouse body weight. When the body weight decrease exceeded 15%, administration was stopped, and when it recovered to within 10%, administration was resumed.

d, After starting administration of the test drugs, tumor measurement and weighing were carried out twice a week.
e, After the end of the experiment, the animals were euthanized.
f, Data were processed using software such as GraphPad Prism. The tumor growth inhibitory efficacy of the compounds was evaluated by TGI (%) and ΔT/ΔC (%). ΔT/ΔC (%) = (T-T0)/(C-C0) × 100, wherein T and C are respectively the tumor volumes of the treatment group and the vehicle control group at the end of the experiment, and T0 and C0 are the tumor volumes of the treatment group and the vehicle control group at the start of the experiment. Tumor growth inhibition rate (TGI) (%) = 100-ΔT/ΔC (%). When tumor regression occurred, tumor growth inhibition rate (TGI) (%) = 100-(T-T0)/T0 × 100

4. Experimental results and conclusion

The tumor-bearing mice tolerated treatments with drug A alone and in combination with drug B or drug C. The drug A + drug B treatment group was significantly superior to each monotherapy group, and the drug A + drug C treatment group was also significantly superior to each monotherapy group. In addition, drug A combined with drug B and drug C treatment group had the most significant tumor growth inhibitory effect, superior to each monotherapy group or two-drug combination groups.

**Example 7.** In vivo pharmacodynamic study of drug A monotherapy or combination therapy in a mouse subcutaneous xenograft tumor model of the human endometrial cancer cell line RL95-2

1. Test drugs

Drug A: prepared by the method in Example 1, with normal saline used for drug preparation.
Drug B: 1'-((7-ethyl-6-oxo-5,6-dihydro-1,5-naphthyridin-3-yl)methyl)-N-methyl-1',2',3',6'-tetrahydro-[3,4'-bipyridine]-6-carboxamide was prepared by the method disclosed in WO2022223025, and was used as drug B.
Drug C: bevacizumab for injection, provided by Suzhou Suncadia Biopharmaceuticals Co., Ltd., with normal saline used for drug preparation.

2. Experimental objective

To evaluate the in vivo efficacy of compound A monotherapy or combination therapy in a nude-mouse subcutaneous xenograft model of the human endometrial cancer cell line RL95-2.

3. Experimental instruments and reagents

3.1 Instruments

Carbon dioxide incubator (HERAcell-240i, Thermo Fisher Scientific)

Precision balance (SECURA225D-1CN, Sartorius Group, Germany)
General balance (HZ2002A, Changzhou Tianzhiping Instrument Equipment Co., Ltd.)
Biosafety cabinet (BSC1300-II-A2, Shandong Xinhua Medical Devices Co., Ltd.)
Digital caliper ((0-150) mm/0.01 mm, Mitutoyo, Japan)
Pipettes (20-200 μL; 100-1000 μL, Eppendorf)

3.2 Reagents

RPMI 1640 was purchased from Gibco, catalog number 22400-071
FBS was purchased from Gibco, catalog number 10091148;
PBS was purchased from Gibco, catalog number 10010023;
Trypsin was purchased from Gibco, catalog number 25200056;
Matrigel was purchased from Corning, catalog number 354234.

4. Experimental procedures and data processing

4.1 Animals

NOD SCID mice, 6-8 weeks old, female, purchased from Jiangsu Jicui Yaokang Biotechnology Co., Ltd.

4.2 Cell culture and preparation of cell suspension

a, One strain of RL95-2 cells was taken out from the cell bank, the cells were revived with RPMI 1640 medium, and the revived cells were placed in a cell culture flask (with the cell type, date, name of the culturing person, etc. marked on the flask wall) and cultured in a $CO_2$ incubator (the incubator temperature was 37°C, and the $CO_2$ concentration was 5%).
b, The cells were passaged once a week, and after passaging, the culture of cells was continued in a $CO_2$ incubator. This process was repeated until the number of cells met the requirements for in vivo pharmacodynamic study.
c, The cultured cells were collected and counted with a fully automatic cell counter, the cells were resuspended with PBS according to the counting result to prepare a cell suspension (density $10 \times 10^7$/mL), an equal volume of Matrigel was added to the cell suspension and mixed uniformly, and the mixture was placed in an ice box for use later. 3.3 Cell inoculation
a, Before inoculation, the nude mice were marked with disposable universal ear tags for rats and mice;
b, At the time of inoculation, the cell suspension was mixed uniformly, 0.2-1 mL of the cell suspension was drawn with a 1 mL syringe and air bubbles were expelled, and then the syringe was placed on an ice pack for use later;
c, The NOD SCID mouse was properly restrained with the left hand, the position on the right back of the nude mouse near the right shoulder (the inoculation site) was disinfected with a 75% alcohol cotton ball, and inoculation was started after 30 seconds;
d, The experimental NOD SCID mice were inoculated in sequence (each mouse was inoculated with 0.1 mL of the cell suspension).

3.4 Tumor measurement, grouping, and administration in tumor-bearing mice

a, According to the tumor growth condition, tumor measurement was carried out on days 20-30 after the inoculation, and the tumor size was calculated;

Tumor volume calculation: tumor volume ($mm^3$) = length (mm) × width (mm) × width (mm)/2

b, According to the body weight and tumor size of the tumor-bearing mice, grouping was carried out by a random grouping method;
c, According to the grouping results, administration of the test drugs was initiated, and the specific doses and regimens are shown in Table 7-1.

**Table 7-1. Administration and grouping**

| Group | Compound | Administration volume (μL/g) [a] | Route of administration | Administration frequency |
|---|---|---|---|---|
| **1** | Vehicle | 10 | *i.v.* | Single dose |
| **2** | Drug A | 10 | *i.v.* | Single dose |
| **3** | Drug B | 10 | *p.o.* | QD × 3w |
| **4** | Drug C | 10 | *i.v.* | BIW × 3w |
| **5** | Drug A + Drug B | 10 + 10 | *i.v.* + *p.o.* | Single dose + QD × 3w |
| **6** | Drug A+ Drug C | 10 + 10 | *i.v.* + *i.v.* | Single dose + BIW × 3w |
| **7** | Drug B+ Drug C | 10 + 10 | *p.o.*+ *i.v.* | QD × 3w + BIW × 3w |
| **8** | Drug A+ Drug B+ Drug C | 10 + 10+ 10 | *i.v.* + *i.v.* + *i.v.* | Single dose +QD × 3w + BIW × 3w |

a. Administration volume: administration was carried out at 10 μL/g based on mouse body weight. When the body weight decrease exceeded 15%, administration was stopped, and when it recovered to within 10%, administration was resumed.

d, After starting administration of the test drugs, tumor measurement and weighing were carried out twice a week.
e, After the end of the experiment, the animals were euthanized.
f, Data were processed using software such as GraphPad Prism. The tumor growth inhibitory efficacy of the compounds was evaluated by TGI (%) and ΔT/ΔC (%). ΔT/ΔC (%) = (T-T0)/(C-C0) × 100, wherein T and C are respectively the tumor volumes of the treatment group and the vehicle control group at the end of the experiment, and T0 and C0 are the tumor volumes of the treatment group and the vehicle control group at the start of the experiment. Tumor growth inhibition rate (TGI) (%) = 100-ΔT/ΔC (%). When tumor regression occurred, tumor growth inhibition rate (TGI) (%) = 100-(T-T0)/T0 × 100

4. Experimental results and conclusion

The tumor-bearing mice tolerated treatments with drug A alone and in combination with drug B or drug C. The drug A + drug B treatment group was significantly superior to each monotherapy group, and the drug A + drug C treatment group was also significantly superior to each monotherapy group. In addition, drug A combined with drug B and drug C treatment group had the most significant tumor growth inhibitory effect, superior to each monotherapy group or two-drug combination groups.

Example 8. Clinical study of a PARP inhibitor in combination with an anti-B7H4 antibody-drug conjugate for treating advanced solid tumors

**1. Study objectives**

- Main study objective:
  To evaluate the safety and tolerability of PARP inhibitor combination treatments in subjects with advanced solid tumors.
- Secondary study objectives:

  1. To evaluate the PK characteristics of PARP inhibitor combination treatments in subjects with advanced solid tumors;
  2. To evaluate other safety endpoints of PARP inhibitor combination treatments in subjects with advanced solid tumors;
  3. To evaluate the efficacy of PARP inhibitor combination treatments in subjects with advanced solid tumors;
  4. To evaluate the PK characteristics of anti-B7H4 antibody-drug conjugate combination treatments in patients with advanced solid tumors;
  5. To evaluate the immunogenicity of the anti-B7H4 antibody-drug conjugate in a combination treatment regimen.

- Exploratory study objectives:

    1. The relationship between exposure and effect;
    2. To explore biomarkers that predict or affect the efficacy of PARP inhibitor combination treatments.

**2. Name of investigational drugs:**

(1) Anti-B7H4 antibody-drug conjugate

Dosage form: injection (lyophilized powder), specification: 50 mg/vial, manufacturer: Shanghai Hansoh Biomedical Technology Co., Ltd.

(2) Poly(ADP-ribose) polymerase (poly adenosinediphosphate ribose polymerase, PARP) inhibitor

1'-((7-ethyl-6-oxo-5,6-dihydro-1,5-naphthyridin-3-yl)methyl)-N-methyl-1',2',3',6'-tetrahydro-[3,4'-bipyridine]-6-carboxamide is prepared by the method disclosed in WO2022223025.

Dosage form: tablet, specification: 10 mg, 40 mg, manufacturer: Shanghai Hansoh Biomedical Technology Co., Ltd.

**3. Target population:**

① Patients confirmed by cytology or histology to have recurrent ovarian cancer, fallopian tube cancer, or primary peritoneal cancer or patients with advanced breast cancer, who have failed or are intolerant to standard treatment and;
② Patients with other advanced solid tumors who have failed or are intolerant to standard treatment and who, as assessed by the investigator, may benefit from the study treatments.

**4. Dosing regimen:**

The dose-escalation stage of this study is set with the following cohorts:
4A: The PARP inhibitor in combination with the anti-B7H4 antibody-drug conjugate

During the dose-escalation period of the poly(ADP-ribose) polymerase inhibitor, administration is carried out at a starting dose of 40 mg QD. Subjects will start continuous oral administration once daily on C1D1 (Cycle 1 Day 1), with each 21 days as one treatment cycle. Subjects should fast from 2 hours before administration of the poly(ADP-ribose) polymerase inhibitor to 1 hour after administration.

During the dose-escalation period of the B7H4 antibody-drug conjugate, administration is carried out at a starting dose of 3.8 mg/kg Q3W, and there are a total of 3 preset dose groups (2.8 mg/kg, 3.8 mg/kg, 4.8 mg/kg Q3W); if the starting dose is not tolerable, it is decreased to the 2.8 mg/kg Q3W dose level. Subjects will, starting from C1D1 (+3 days), receive intravenous administration of the B7H4 antibody-drug conjugate, with each 21 days as one treatment cycle; starting from C2 (Cycle 2), the interval between the date of each subsequent first intravenous administration and the date of the previous first intravenous administration is 21 (±3) days.

**5. Study endpoints:**

- Primary study endpoint:
  Maximum tolerated dose (MTD) or maximum applicable dose (MAD) of PARP inhibitor combination treatments.
- Secondary study endpoints:

    1. PK characteristics of PARP inhibitor combination treatments;
    2. Safety of PARP inhibitor combination treatments;
    3. Efficacy of PARP inhibitor combination treatments (investigator assessed):

        a. Objective response rate (ORR), disease control rate (DCR), and duration of response (DoR) as assessed by the investigator according to the RECIST v1.1 criteria, applicable to subjects with target lesions at baseline (all solid tumors except prostate cancer);

b. Progression-free survival (PFS) as assessed by the investigator according to the RECIST v1.1 criteria (all solid tumors except prostate cancer);
c. Overall survival (OS);
d. ORR as assessed by the investigator according to RECIST v1.1 and GCIG CA-125 criteria (ovarian cancer only);
e. ORR, DCR, DoR, and radiographic progression-free survival (rPFS) as assessed by the investigator according to RECIST v1.1 (soft tissue) and PCWG3 criteria (bone lesions) (prostate cancer only);
f. The proportion of subjects with a CA-125 decrease of ≥ 50% relative to baseline and the time to CA-125 progression (ovarian cancer only);
g. PSA50 response rate and time to PSA progression (prostate cancer only).

6. PK characteristics of treatments in combination with the anti-B7H4 antibody-drug conjugate in patients with advanced solid tumors.

- Exploratory study endpoints:

1. To explore the relationship between exposure and effect;
2. To explore biomarkers that predict or affect the efficacy of PARP inhibitor combination treatments.

**Example 9.** Clinical study of the PARP inhibitor in combination with the anti-B7H4 antibody-drug conjugate and bevacizumab for treating advanced solid tumors

**1. Study objectives**

- Main study objective:
To evaluate the safety and tolerability of PARP inhibitor combination treatments in subjects with advanced solid tumors.
- Secondary study objectives:

1. To evaluate the PK characteristics of PARP inhibitor combination treatments in subjects with advanced solid tumors;
2. To evaluate other safety endpoints of PARP inhibitor combination treatments in subjects with advanced solid tumors;
3. To evaluate the efficacy of PARP inhibitor combination treatments in subjects with advanced solid tumors;
4. To evaluate the PK characteristics of anti-B7H4 antibody-drug conjugate combination treatments in patients with advanced solid tumors;
5. To evaluate the immunogenicity of the anti-B7H4 antibody-drug conjugate in a combination treatment regimen.

- Exploratory study objectives:

1. The relationship between exposure and effect;
2. To explore biomarkers that predict or affect the efficacy of PARP inhibitor combination treatments.

**2. Name of investigational drugs:**

(1) Anti-B7H4 antibody-drug conjugate

Dosage form: injection (lyophilized powder), specification: 50 mg/vial, manufacturer: Shanghai Hansoh Biomedical Technology Co., Ltd.

(2) Poly(ADP-ribose) polymerase (poly adenosinediphosphate ribose polymerase, PARP) inhibitor

1'-((7-ethyl-6-oxo-5,6-dihydro-1,5-naphthyridin-3-yl)methyl)-N-methyl-1',2',3',6'-tetrahydro-[3,4'-bipyridine]-6-carboxamide is prepared by the method disclosed in WO2022223025.
Dosage form: tablet, specification: 10 mg, 40 mg, manufacturer: Shanghai Hansoh Biomedical Technology Co., Ltd.

(3) Anti-VEGF antibody

Bevacizumab injection, dosage form: sterile solution for injection, specification: 100 mg (4 mL)/vial, manufacturer: Suzhou Suncadia Biopharmaceuticals Co., Ltd.

(4) Platinum drugs

Carboplatin injection, characteristics: carboplatin for injection is a white or off-white lyophilized loose mass or powder, manufacturer: Qilu Pharmaceutical Co., Ltd.

Cisplatin injection, characteristics: a clear liquid that is light yellow-green to light yellow to slightly viscous, manufacturer: Jiangsu Hansoh Pharmaceutical Group Co., Ltd.

**3. Target population:**

Patients with platinum-sensitive recurrent ovarian cancer:

① Histologically confirmed high-grade (poorly differentiated) serous or high-grade (poorly differentiated) endometrioid epithelial ovarian cancer, fallopian tube cancer, or primary peritoneal cancer.
② Disease progression or recurrence after 2-3 lines of treatment, and the time from disease progression or recurrence to completion of the last platinum-containing chemotherapy is ≥ 6 months.
③ No systemic antitumor therapy has been received after the most recent progression or recurrence.
④ Previously received treatment with at least 1 PARP inhibitor.
⑤ As assessed by the investigator, not suitable for cytoreductive surgery (for example, satisfactory cytoreduction cannot be achieved by surgical resection), or the subject refuses cytoreductive surgery.

Newly diagnosed advanced ovarian cancer without prior systemic antitumor treatment:

① Histologically or cytologically confirmed, or with the main histological component being high-grade (poorly differentiated) serous or high-grade (poorly differentiated) endometrioid ovarian cancer, fallopian tube cancer, or primary peritoneal cancer, with FIGO stage III or IV. Histological types of carcinosarcoma, undifferentiated, or mucinous carcinoma and clear cell carcinoma among epithelial ovarian cancers are not allowed.
② The subject has completed or plans to undergo initial tumor cytoreductive surgery, or plans to undergo interval tumor cytoreductive surgery.
③ Has not previously received PARP inhibitor treatment.
④ Has not previously received any systemic antitumor treatment for advanced ovarian cancer.

**4. Dosing regimen:**

The dose-escalation stage of this study is set with the following cohorts:
Cohort 5A: The anti-B7H4 antibody-drug conjugate in combination with the PARP inhibitor and bevacizumab.

During this dose-escalation process, the induction phase uses the anti-B7H4 antibody-drug conjugate in combination with bevacizumab and a platinum drug, and the sequential maintenance phase uses the anti-B7H4 antibody-drug conjugate in combination with bevacizumab and the PARP inhibitor.

During the induction treatment phase, the day of the subject's first administration is recorded as C1D1 (Cycle 1 Day 1), and the day of each subsequent administration is D1 of the next treatment cycle, and the interval window period for administration is 21±3 days; after completing the induction treatment phase and before entering maintenance treatment, the subject also needs to undergo 1 tumor imaging assessment, and may enter the maintenance treatment phase only if the disease has not progressed. The first administration of the PARP inhibitor during the maintenance treatment phase should occur within 3-6 weeks after the last administration of the platinum drug during the induction treatment phase. The date on which the PARP inhibitor drug treatment is first received during the maintenance treatment phase is defined as D1 of that visit, and thereafter each 21 days is one treatment cycle. The following are the dosing regimens for each type of drug in this cohort:
The starting dose of the PARP inhibitor is 40 mg QD, with a total of 3 preset dose groups (20 mg, 40 mg, 80 mg QD); if the starting dose is not tolerable, it is decreased to the 20 mg QD dose level. The PARP inhibitor treatment is started within 3-6 weeks after the end of platinum chemotherapy in the induction phase, with each 21 days as one treatment cycle, and continuous oral administration once daily. Subjects should fast from 2 hours before administration of the PARP inhibitor to 1 hour after administration.

The starting dose of the anti-B7H4 antibody-drug conjugate is 3.8 mg/kg Q3W, with a total of 3 preset dose groups

(2.8 mg/kg, 3.8 mg/kg, 4.8 mg/kg Q3W); if the starting dose is not tolerable, it is decreased to the 2.8 mg/kg Q3W dose level. Subjects will, starting from C1D1 (+3 days), receive intravenous administration of the anti-B7H4 antibody-drug conjugate, and starting from C2, the interval between the date of each subsequent first intravenous administration and the date of the previous first intravenous administration is 21 (±3) days.

Platinum drugs: according to the investigator's choice, cisplatin 75 mg/m$^2$ Q3W or carboplatin AUC 5 mg/mL/min Q3W is administered, subjects with platinum-sensitive recurrent ovarian cancer receive 6 cycles of platinum chemotherapy, and subjects with newly diagnosed advanced ovarian cancer receive 6-8 cycles of platinum chemotherapy. Subjects will, starting from C1D1 (+3 days), receive intravenous administration of the platinum drug, and starting from C2, the interval between the date of each subsequent first intravenous administration and the date of the previous first intravenous administration is 21 (±3) days. The investigator may choose cisplatin or carboplatin according to the specific condition of the subject, and during treatment, interchange use of cisplatin and carboplatin due to tolerability issues is allowed, but the subject should be fully informed of the risk of cross-allergy. If the subject cannot tolerate it for safety reasons, treatment with that drug is terminated.

Bevacizumab: all subjects in the dose-escalation stage are required to receive the bevacizumab treatment. Subjects will receive intravenous administration at 15 mg/kg Q3W. Subjects with newly diagnosed ovarian cancer will receive bevacizumab starting from C2D1 (+3 days), with continuous administration for up to 15 months; subjects with platinum-sensitive recurrent ovarian cancer will start administration from C1D1 (+3 days), with continuous administration until objective disease progression (excluding continued treatment after disease progression) or until other treatment discontinuation criteria are met.

For this cohort, it is recommended that intravenous drugs are administered after administration of the PARP inhibitor, and that the intravenous drugs are administered in the following order: bevacizumab first (if applicable), then the anti-B7H4 antibody-drug conjugate, and finally the platinum drug (if applicable). All drugs are continuously administered until completion of the preset treatment course or until objective disease progression (excluding continued treatment after disease progression) or until other treatment discontinuation criteria are met.

**5. Study endpoints:**

- Primary study endpoint:
  Maximum tolerated dose (MTD) or maximum applicable dose (MAD) of PARP inhibitor combination treatments.
- Secondary study endpoints:

  1. PK characteristics of PARP inhibitor combination treatments;
  2. Safety of PARP inhibitor combination treatments;
  3. Efficacy of PARP inhibitor combination treatments (investigator assessed):

     a. Objective response rate (ORR), disease control rate (DCR), and duration of response (DoR) as assessed by the investigator according to the RECIST v1.1 criteria, applicable to subjects with target lesions at baseline (all solid tumors except prostate cancer);
     b. Progression-free survival (PFS) as assessed by the investigator according to the RECIST v1.1 criteria (all solid tumors except prostate cancer);
     c. Overall survival (OS);
     d. ORR as assessed by the investigator according to RECIST v1.1 and GCIG CA-125 criteria (ovarian cancer only);
     e. ORR, DCR, DoR, and radiographic progression-free survival (rPFS) as assessed by the investigator according to RECIST v1.1 (soft tissue) and PCWG3 criteria (bone lesions) (prostate cancer only);
     f. The proportion of subjects with a CA-125 decrease of ≥ 50% relative to baseline and the time to CA-125 progression (ovarian cancer only);
     g. PSA50 response rate and time to PSA progression (prostate cancer only).

  6. PK characteristics of treatments in combination with the anti-B7H4 antibody-drug conjugate in patients with advanced solid tumors.

- Exploratory study endpoints:

  1. To explore the relationship between exposure and effect;
  2. To explore biomarkers that predict or affect the efficacy of PARP inhibitor combination treatments.

## Claims

1. Use of an antibody-drug conjugate in combination with a poly(ADP-ribose) polymerase inhibitor in the preparation of a medication for treating cancer, wherein the structure of the antibody-drug conjugate is as shown in formula (I):

(I)

wherein:

n is 1 to 10, preferably 2 to 8, more preferably 3 to 8, and n is a decimal or an integer;
Pc is an anti-B7H4 antibody or an antigen-binding fragment thereof.

2. The use according to claim 1, **characterized in that** the anti-B7H4 antibody or an antigen-binding fragment thereof comprises: heavy-chain HCDR1, HCDR2, and HCDR3 respectively as shown in the amino acid sequences of SEQ ID NO: 01, 02, and 03, and light-chain LCDR1, LCDR2, and LCDR3 respectively as shown in the amino acid sequences of SEQ ID NO: 04, 05, and 06.

3. The use according to claim 1 or 2, **characterized in that** the anti-B7H4 antibody or an antigen-binding fragment thereof is selected from a humanized antibody or a fragment thereof.

4. The use according to claim 3, **characterized in that** the anti-B7H4 antibody or an antigen-binding fragment thereof comprises a heavy-chain constant region of a human IgG1, IgG2, IgG3, or IgG4 isotype, and a light-chain constant region comprising $\kappa$ or $\lambda$; preferably, the anti-B7H4 antibody or the antigen-binding fragment thereof comprises a heavy-chain constant region of an IgG1 or IgG4 isotype.

5. The use according to claim 3, **characterized in that** the heavy-chain variable region sequence of the anti-B7H4 antibody or an antigen-binding fragment thereof is the sequence as shown in SEQ ID NO: 07 or a variant thereof, and the light-chain variable region sequence is the sequence as shown in SEQ ID NO: 08 or a variant thereof.

6. The use according to any one of claims 1 to 5, **characterized in that** the heavy-chain sequence of the anti-B7H4 antibody or an antigen-binding fragment thereof is the sequence as shown in SEQ ID NO: 09 or a variant thereof, and the light-chain sequence is the sequence as shown in SEQ ID NO: 10 or a variant thereof.

7. The use according to any one of claims 1 to 6, **characterized in that** the poly(ADP-ribose) polymerase inhibitor is selected from one or more of Olaparib, Fluzoparib, Niraparib, Pamiparib, Rucaparib, Talazoparib, Veliparib, Senaparib, CEP-8983, BGB-290, or 1'-((7-ethyl-6-oxo-5,6-dihydro-1,5-naphthyridin-3-yl)methyl)-N-methyl-1',2',3',6'-tetrahydro-[3,4'-bipyridine]-6-carboxamide.

8. The use according to any one of claims 1-7, wherein the combination has additive or synergistic effects.

9. The use according to any one of claims 1 to 8, wherein the antibody-drug conjugate and the poly(ADP-ribose) polymerase inhibitor are respectively comprised as active ingredients in different formulations and are administered simultaneously or sequentially.

10. The use according to any one of claims 1 to 8, **characterized in that** the antibody-drug conjugate and the poly(ADP-ribose) polymerase inhibitor are comprised as active ingredients in a single formulation and administered.

11. The use according to any one of claims 1-10, further in combination with an anti-VEGF antibody.

**12.** The use according to claim 11, wherein the anti-VEGF antibody is selected from Bevacizumab, Ranibizumab, Sevacizumab, Suvemcitug, Varisacumab, CMAB-801, and LYN-00101.

**13.** The use according to claim 11 or 12, wherein the antibody-drug conjugate, the poly(ADP-ribose) polymerase inhibitor, and the anti-VEGF antibody are respectively comprised as active ingredients in different formulations and are administered simultaneously, sequentially, continuously, alternately, or separately.

**14.** The use according to any one of claims 11 to 13, wherein the administration dose of the anti-VEGF antibody is 1.0 mg/kg to 100 mg/kg, preferably 1.0 mg/kg to 40 mg/kg, more preferably 1.0 mg/kg to 30 mg/kg, and the administration frequency is once every week, once every two weeks, once every three weeks, or once every four weeks.

**15.** The use according to any one of claims 1 to 14, **characterized in that** the dose of the antibody-drug conjugate is 0.1 mg/kg to 20.0 mg/kg, preferably 1.0 mg/kg to 15.0 mg/kg; the administration frequency is once every week, once every two weeks, once every three weeks, or once every four weeks.

**16.** The use according to any one of claims 1-15, **characterized in that** the administration dose of the poly(ADP-ribose) polymerase inhibitor is 1-500 mg/kg, preferably 10 mg/kg, 15 mg/kg, 20 mg/kg, 25 mg/kg, 30 mg/kg, 35 mg/kg, 40 mg/kg, 45 mg/kg, 50 mg/kg, 55 mg/kg, 60 mg/kg, 65 mg/kg, 70 mg/kg, 75 mg/kg, 80 mg/kg, 85 mg/kg, 90 mg/kg, 95 mg/kg, 100 mg/kg, 110 mg/kg, 120 mg/kg, 130 mg/kg, 140 mg/kg, 150 mg/kg, 160 mg/kg, 170 mg/kg, 180 mg/kg, 190 mg/kg, 200 mg/kg, 210 mg/kg, 220 mg/kg, 230 mg/kg, 240 mg/kg, 250 mg/kg, 260 mg/kg, 270 mg/kg, 280 mg/kg, 290 mg/kg, 300 mg/kg, 320 mg/kg, 340 mg/kg, 350 mg/kg, 360 mg/kg, 380 mg/kg, 400 mg/kg, 420 mg/kg, 440 mg/kg, 450 mg/kg, 460 mg/kg, 480 mg/kg, or 500 mg/kg; the administration frequency is once daily, twice daily, three times daily, once every two days, or once every three days.

**17.** The use according to any one of claims 1 to 16, **characterized in that** the cancer is a solid tumor; preferably, the cancer is selected from uterine cancer, breast cancer, ovarian cancer, pancreatic cancer, cholangiocarcinoma, fallopian tube cancer, gastric cancer, colorectal cancer, primary peritoneal cancer, prostate cancer, and lung cancer; more preferably it is selected from uterine cancer, breast cancer, and ovarian cancer.

**18.** The use according to any one of claims 1 to 17, **characterized in that** the cancer is selected from cervical cancer, endometrial cancer, triple-negative breast cancer, ovarian epithelial cancer, epithelial ovarian cancer, platinum-sensitive recurrent ovarian cancer, intrahepatic cholangiocarcinoma, and extrahepatic cholangiocarcinoma; preferably it is selected from endometrial cancer, triple-negative breast cancer, and ovarian epithelial cancer; further preferably, the ovarian epithelial cancer is selected from high-grade serous ovarian cancer, low-grade serous ovarian cancer, endometrioid ovarian cancer, mucinous ovarian cancer, and ovarian clear cell carcinoma.

**19.** A pharmaceutical composition containing the antibody-drug conjugate and the poly (ADP-ribose) polymerase inhibitor described in any one of claims 1-18, **characterized in that** it comprises one or more pharmaceutically acceptable excipients, diluents, or carriers.

**20.** A pharmaceutical combination, comprising the antibody-drug conjugate as defined in any one of claims 1-18 and a pharmaceutically acceptable excipient thereof, and the poly (ADP-ribose) polymerase inhibitor as defined in any one of claims 1 to 18 and a pharmaceutically acceptable excipient thereof.

**21.** A pharmaceutical composition containing the antibody-drug conjugate, the poly (ADP-ribose) polymerase inhibitor, and the anti-VEGF antibody described in any one of claims 11-18, **characterized in that** it comprises one or more pharmaceutically acceptable excipients, diluents, or carriers.

**22.** A pharmaceutical combination, comprising the antibody-drug conjugate as defined in any one of claims 1-18 and a pharmaceutically acceptable excipient thereof, the poly (ADP-ribose) polymerase inhibitor as defined in any one of claims 1 to 18 and a pharmaceutically acceptable excipient thereof, and the anti-VEGF antibody as defined in any one of claims 11-18 and a pharmaceutically acceptable excipient thereof.

**23.** A method for treating and/or preventing cancer, comprising: administering in combination to a patient an effective amount of the antibody-drug conjugate as defined in any one of claims 1 to 18, the poly(ADP-ribose) polymerase inhibitor, and optionally the anti-VEGF antibody as defined in any one of claims 11 to 18, wherein the combination administration may be simultaneous, parallel, sequential, continuous, alternating, or separate administration.

**24.** A method for treating and/or preventing cancer, the method comprising administering in combination to a patient, during the induction treatment stage, the antibody-drug conjugate as defined in any one of claims 1 to 18, the anti-VEGF antibody as defined in any one of claims 11 to 18, and a platinum drug, wherein the combination administration may be simultaneous, parallel, sequential, continuous, alternating, or separate administration, and during the maintenance treatment stage after induction treatment, administering in combination to the patient an effective amount of the antibody-drug conjugate as defined in any one of claims 1 to 18, the poly(ADP-ribose) polymerase inhibitor, and the anti-VEGF antibody as defined in any one of claims 11 to 18, wherein the combination administration may be simultaneous, parallel, sequential, continuous, alternating, or separate administration.

**25.** The method according to claim 24, wherein the platinum drug is selected from carboplatin, cisplatin, oxaliplatin, nedaplatin (Nedaplatin), lobaplatin (Lobaplatin), satraplatin (Satraplatin), cycloplatin (Cycloplatin), miboplatin (Miboplatin), Enloplatin, Iproplatin, and Dicycloplatin; preferably carboplatin and/or cisplatin.

**26.** The method according to claims 23 to 25, **characterized in that** the cancer is a solid tumor; preferably, the cancer is selected from uterine cancer, breast cancer, ovarian cancer, pancreatic cancer, cholangiocarcinoma, fallopian tube cancer, gastric cancer, colorectal cancer, primary peritoneal cancer, prostate cancer, and lung cancer; more preferably it is selected from uterine cancer, breast cancer, and ovarian cancer.

**27.** The method according to claims 23 to 26, **characterized in that** the cancer is selected from cervical cancer, endometrial cancer, triple-negative breast cancer, ovarian epithelial cancer, platinum-sensitive recurrent ovarian cancer, intrahepatic cholangiocarcinoma, and extrahepatic cholangiocarcinoma; preferably it is selected from endometrial cancer, triple-negative breast cancer, and ovarian epithelial cancer; further preferably, the ovarian epithelial cancer is selected from high-grade serous ovarian cancer, low-grade serous ovarian cancer, high-grade endometrioid epithelial ovarian cancer, endometrioid ovarian cancer, mucinous ovarian cancer, and ovarian clear cell carcinoma.

N=1
Drug B [nM]
Drug A [nM]
Synergistic effect
Antagonistic effect
Bliss model combination index
OVCAR-3
SK-OV-3
OAW42
KURAMOCHI
CAOV3
COV362
OAW28


Fig. 1

N=1
Drug B [nM]
Drug A [nM]
Synergistic effect
Antagonistic effect
Bliss model combination index
HCC38
MX-1
CAL-51
CAL-148


Fig. 2

N=1
Drug B [nM]
Drug A [nM]
Synergistic effect
Antagonistic effect
Bliss model combination index
RL95-2
HEC-1A
EN
Ishikawa
AN3CA
EFE-184
MFE280
KLE


Fig. 3

MX-1 Xenograft
Tumor volume (mm³, Mean ± SEM)
Time after administration (days)
G1. Vehicle control (0.5% HPMC), p.o. QD
G2. Drug B, 0.3 mg/kg, p.o. QD
G3. Drug A 1 mg/kg, i.v. Single dose
G4. Drug A 2 mg/kg, i.v. Single dose
G5. Drug B, 0.3 mg/kg + Drug A, 1 mg/kg
G6. Drug B, 0.3 mg/kg + Drug A, 2 mg/kg

Fig. 4

MX-1 Xenograft
Body weight change (%, Mean ± SEM)
Time after administration (days)
G1. Vehicle control (0.5% HPMC), p.o. QD
G2. Drug B, 0.3 mg/kg, p.o. QD
G3. Drug A 1 mg/kg, i.v. Single dose
G4. Drug A 2 mg/kg, i.v. Single dose
G5. Drug B, 0.3 mg/kg + Drug A, 1 mg/kg
G6. Drug B, 0.3 mg/kg + Drug A, 2 mg/kg

Fig. 5

OV0243 Xenograft
Tumor volume (mm³, Mean ± SEM)
Time after administration (days)
G1. Vehicle control (0.5% HPMC), p.o. QD
G2. Drug B, 0.1 mg/kg, p.o. QD
G3. Drug B 1 mg/kg, p.o. QD
G4. Drug A 5 mg/kg, i.v. Single dose
G5. Drug B, 0.1 mg/kg + Drug A, 5 mg/kg
G6. Drug B, 1 mg/kg + Drug A, 5 mg/kg

Fig. 6

OV0243 Xenograft
Body weight change (%, Mean ± SEM)
30
20
10
0
-10
0 5 10 15 20 25 30 35 40 45 50 55 60 65 70 75 80 85
Time after administration (days)
G1. Vehicle control (0.5% HPMC), p.o. QD
G2. Drug B, 0.1 mg/kg, p.o. QD
G3. Drug B 1 mg/kg, p.o. QD
G4. Drug A 5 mg/kg, i.v. Single dose
G5. Drug B, 0.1 mg/kg + Drug A, 5 mg/kg
G6. Drug B, 1 mg/kg + Drug A, 5 mg/kg

Fig. 7

RL95-2 xenograft
G1. Vehicle control (0.5% HPMC), p.o. QD
G2. Drug B, 1 mg/kg, p.o. QD
G3. Drug A 3 mg/kg, i.v. Single dose
G4. Drug B 1 mg/kg + Drug A, 3 mg/kg
Tumor volume (mm3, Mean ± SEM)
1500
1000
500
0
****
****
0
7
14
21
28
Time after administration (days)

Fig. 8

RL95-2 xenograft
G1. Vehicle control (0.5% HPMC), p.o. QD
G2. Drug B, 1 mg/kg, p.o. QD
G3. Drug A 3 mg/kg, i.v. Single
G4. Drug B 1 mg/kg + Drug A, 3 mg/kg
Body weight change (%, Mean ± SEM)
20
10
0
-10
0
7
14
21
28
Time after administration (days)

Fig. 9

**INTERNATIONAL SEARCH REPORT**

International application No. **PCT/CN2024/132015**

**A. CLASSIFICATION OF SUBJECT MATTER**

A61K47/68(2017.01)i; A61K31/4375(2006.01)i; C07D401/14(2006.01)i; C07D471/04(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 江苏豪森药业集团有限公司, 上海翰森生物医药科技有限公司, 常州恒邦药业有限公司, 抗体药物偶联物, conjugate, ADC, B7H4, B7-H4, 多腺苷二磷酸核糖聚合酶, PARP, 奥拉帕尼, Olaparib, 氟唑帕利, Fluzoparib, 尼拉帕利, Niraparib, 帕米帕利, Pamiparib, 鲁卡帕利, Rucaparib, 他拉唑帕尼, Talazoparib, 维利帕尼, Veliparib, 塞纳帕利, Senaparib, CEP-8983, BGB-290, 贝伐珠, Bevacizumab, 雷珠, Ranibizumab, 赛伐珠, Sevacizumab, 苏维西塔, Suvemcitug , 伐利苏, Varisacumab, CMAB-801, LYN-00101

**C. DOCUMENTS CONSIDERED TO BE RELEVANT**

| Category* | Citation of document, with indication, where appropriate, of the relevant passages | Relevant to claim No. |
|---|---|---|
| Y | WO 2023156434 A1 (MEDIMMUNE LTD.) 24 August 2023 (2023-08-24) abstract, claims 1-6, and description, examples 1-4 | 1-27 |
| Y | WO 2020244657 A1 (SHANGHAI HANSOH BIOMEDICAL CO., LTD. et al.) 10 December 2020 (2020-12-10) description, page 78, lines 1-18, page 80, lines 16-26, and page 81, lines 1-6, embodiment 9, and claims 35 and 39-41 | 1-27 |
| Y | WO 2022223025 A1 (SHANGHAI HANSOH BIOMEDICAL CO., LTD. et al.) 27 October 2022 (2022-10-27) abstract, claims 1-22, and description, embodiment 1, and test example 1 | 7-27 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 February 2025** | **20 February 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/132015**

**C. DOCUMENTS CONSIDERED TO BE RELEVANT**

| Category* | Citation of document, with indication, where appropriate, of the relevant passages | Relevant to claim No. |
|---|---|---|
| Y | 孙芩玲等 (SUN, Qinling et al.). "PARP抑制剂与肿瘤单克隆抗体联用的疗效及机制 (The Mechanism and Effects of Combination Therapy of PARP Inhibitors and Moloclonal Antibodies)" *肿瘤药学 (Anti-tumor Pharmacy),* Vol. 6, No. (6), 31 December 2016 (2016-12-31), pp. 401-406 | 11-27 |
| Y | WO 2020238932 A1 (JIANGSU HENGRUI MEDICINE CO., LTD.) 03 December 2020 (2020-12-03) abstract, and claims 1-21 | 11-27 |
| Y | WO 2018099423 A1 (JIANGSU HENGRUI MEDICINE CO., LTD.) 07 June 2018 (2018-06-07) abstract, and claims 1-16 | 11-27 |
| A | WO 2022188832 A1 (AKESO BIOPHARMA, INC.) 15 September 2022 (2022-09-15) abstract, and claims 1-29 | 1-27 |

Form PCT/ISA/210 (second sheet) (July 2022)

INTERNATIONAL SEARCH REPORT

International application No.
**PCT/CN2024/132015**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **23-27**
because they relate to subject matter not required to be searched by this Authority, namely:

Claims 23-27 relate to a method for treating and/or preventing cancer, which falls within subject matter for which no search is required by the International Searching Authority as defined in PCT Rule 39.1(iv). A search is performed on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/132015**

| Patent document cited in search report | Publication date (day/month/year) | Patent family member(s) | Publication date (day/month/year) |
|---|---|---|---|
| WO 2023156434 A1 | 24 August 2023 | None | |
| WO 2020244657 A1 | 10 December 2020 | EP 3981434 A1 | 13 April 2022 |
| | | EP 3981434 A4 | 01 November 2023 |
| | | AU 2020288275 A1 | 03 February 2022 |
| | | US 2023072897 A1 | 09 March 2023 |
| | | KR 20220017946 A | 14 February 2022 |
| | | BR 112021024406 A2 | 19 April 2022 |
| | | JP 2022535858 A | 10 August 2022 |
| | | TW 202112398 A | 01 April 2021 |
| | | MX 2021014960 A | 04 March 2022 |
| | | CA 3142641 A1 | 10 December 2020 |
| WO 2022223025 A1 | 27 October 2022 | EP 4328225 A1 | 28 February 2024 |
| | | JP 2024514338 A | 01 April 2024 |
| | | TW 202309026 A | 01 March 2023 |
| | | CA 3215823 A1 | 27 October 2022 |
| | | BR 112023019797 A2 | 07 November 2023 |
| | | MX 2023012054 A | 23 October 2023 |
| | | AU 2022261029 A1 | 19 October 2023 |
| | | US 2024228490 A1 | 11 July 2024 |
| | | IL 307824 A | 01 December 2023 |
| | | KR 20240005756 A | 12 January 2024 |
| WO 2020238932 A1 | 03 December 2020 | TW 202110448 A | 16 March 2021 |
| WO 2018099423 A1 | 07 June 2018 | EP 3549608 A1 | 09 October 2019 |
| | | EP 3549608 A4 | 12 August 2020 |
| | | TW 201821107 A | 16 June 2018 |
| | | TWI 771344 B | 21 July 2022 |
| | | US 2019275021 A1 | 12 September 2019 |
| | | US 11000518 B2 | 11 May 2021 |
| WO 2022188832 A1 | 15 September 2022 | EP 4190819 A1 | 07 June 2023 |
| | | EP 4190819 A4 | 14 August 2024 |
| | | JP 2024509018 A | 29 February 2024 |
| | | CA 3193662 A1 | 15 September 2022 |
| | | AU 2022233411 A1 | 14 September 2023 |
| | | BR 112023018457 A2 | 10 October 2023 |
| | | IL 305805 A | 01 November 2023 |
| | | MX 2023010681 A | 22 September 2023 |
| | | KR 20230156910 A | 15 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020244657 A
- WO 2022223025 A


### Non-patent literature cited in the description


- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, 1987
- **WARD et al.** *Nature*, 1989, vol. 341, 544-546
- **DI VEROLI et al.** *Bioinformatics*, 2016, vol. 32 (18), 2866-2868